# EUROPÄISCHE PATENTSCHRIFT

(11) **EP 1 682 527 B1**
(45) Veröffentlichungstag und Bekanntmachung des Hinweises auf die Patenterteilung: **24.08.2011**
(21) Anmeldenummer: 04797735.0
(22) Anmeldetag: 09.11.2004
(51) Int. Cl.: C07D 309/30

(54) **VERFAHREN ZUR HERSTELLUNG VON STATINEN**
METHOD FOR THE PRODUCTION OF STATINS
PROCEDE DE FABRICATION DE STATINES

(30) Priorität: 11.11.2003 DE 10352659
(43) Veröffentlichungstag der Anmeldung: 26.07.2006
(73) Patentinhaber: Ratiopharm GmbH, 89079 Ulm (DE)
(72) Erfinder: TARAROV, Vitali, Moskau (RU); KÖNIG, Gerd, 08056 Zwickau (DE); BÖRNER, Armin, 18059 Rostock (DE)
(74) Vertreter: Best, Michael
(86) Internationale Anmeldenummer: PCT/EP2004/012659
(87) Internationale Veröffentlichungsnummer: WO 2005/047276

(56) Entgegenhaltungen:
- EP-A- 0 583 171
- WO-A-02/06266
- US-A- 4 625 039
- US-B1- 6 417 374
- PRASAD K. ET AL.: "Asymmetric Synthesis of (3R-trans)- and (3S-cis)hydroxy-5-pentanolides" TETRAHEDRON LETTERS, Bd. 25, Nr. 32, 1984, Seiten 3391-3394, XP002317576 in der Anmeldung erwähnt
- BENNETT F ET AL: "METHYL (3R)-3-HYDROXYHEX-5-ENOATE" JOURNAL OF THE CHEMICAL SOCIETY, PERKIN TRANSACTIONS 1, CHEMICAL SOCIETY. LETCHWORTH, GB, Bd. 1, 1991, Seiten 133-140, XP002178272 ISSN: 0300-922X in der Anmeldung erwähnt
- WATANABE M ET AL: "SYNTHESIS AND BIOLOGICAL ANTIVITY OF METHANESULFONAMIDE PYRIMIDINE-AND N-METHANESULFONYL PYRROLE-SUBSTITUTED 3,5-DIHYDROXY-6-HEPTENOATES, A NOVEL SERIES OF HMG-COA REDUCTASE INHIBITORS" BIOORGANIC & MEDICINAL CHEMISTRY, ELSEVIER SCIENCE LTD, GB, Bd. 5, Nr. 2, 1997, Seiten 437-444, XP000882043 ISSN: 0968-0896
- ROSEN T ET AL: "SYNTHETIC AND BIOLOGICAL STUDIES OF COMPACTIN AND RELATED COMPOUNDSSYNTHESIS OF THE LACTONE MOIETY OF COMPACTIN" JOURNAL OF ORGANIC CHEMISTRY, AMERICAN CHEMICAL SOCIETY. EASTON, US, Bd. 49, 19. Oktober 1994 (1994-10-19), Seiten 3994-4003, XP000996454 ISSN: 0022-3263 in der Anmeldung erwähnt
- GHOSH A. K. ET AL.: "Chelation-Controlled Reduction" J. ORG. CHEM., Bd. 67, 2002, Seiten 8783-8788, XP002317577

## Beschreibung

Die vorliegende Erfindung betrifft ein Verfahren zur Herstellung von Statinen, die als HMG-CoA-Reduktaseinhibitoren bekannt sind. Einige der Zwischenverbindungen zur Verwendung in dem erfindungsgemäßen Verfahren sind neue Verbindungen.

Statine sind eine bekannte Klasse von Wirkstoffen, die das Enzym Hydroxymethylglutaryl(HMG)-CoA-Reduktase hemmen. Die Wirkstoffe sind weit verbreitet, insbesondere als Cholesterinsenker im Blut. Bekannte Statine sind beispielsweise Cerivastatin, Fluvastatin, Itavastatin, BMY22089, Rosuvastatin, Glenvastatin und Atorvastätin. Synthesewege für die Statine sind bekannt und in einer Vielzahl von Druckschriften beschrieben. Statine sind im Prinzip aufgebaut aus einem aromatischen, heterocyclischen oder einem aromatisch-heterocyclischen, substituierten oder unsubstituierten, mono-, di- oder polycyclischen Ringsystem, an dem die sogenannte Statinseitenkette entweder in der offenkettigen Form oder in der Lactonform hängt 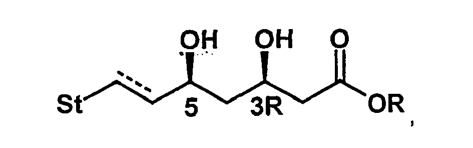 wobei St das vorstehend beschriebene Ringsystem, also den Rest des Statins, darstellt. Unter den Begriff "Statin", wie er im Rahmen dieser Beschreibung verwendet wird, werden auch die pharmazeutisch verträglichen Salze, Hydrate, Solvate, Ester und Ether der im Stand der Technik beschriebenen Statine verstanden.

Von entscheidender Bedeutung für die Wirksamkeit der Statine ist die räumliche Anordnung der Hydroxylgruppen in der Statinseitenkette wie sie in der vorstehenden Formel angegeben ist. Ökonomisch ist es sinnvoll, bei der Synthese der Statine die Stereochemie bereits in einem sehr frühen Schritt festzulegen und die weiteren Schritte unter Erhalt der Stereochemie, also stereoselektiv, durchzuführen, um möglichst hohe Ausbeuten an dem Endprodukt zu erhalten (Produkte mit einer anderen Stereochemie müssen abgetrennt werden).

Verfahren zur Herstellung von Statinen sind seit langem bekannt und Gegenstand der chemischen Forschung. In einer frühen Veröffentlichung von 1984 (J. Org. Chem. Vol. 49, No. 21, 1984, 3994-4003) wird unter anderem folgende Umsetzung beschrieben 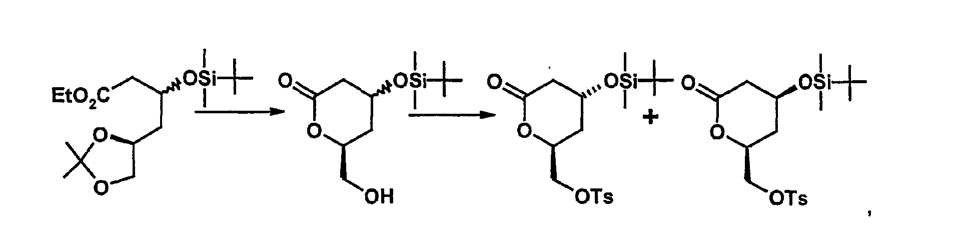 in der Ts eine Tosylschutzgruppe darstellt. Die Verbindung 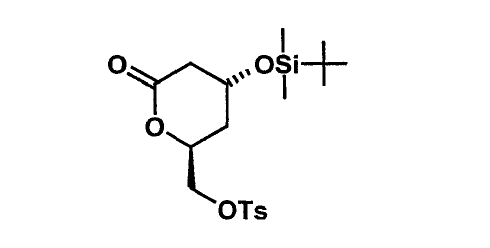 wird als mögliches Zwischenprodukt für die Herstellung des Lactonrestes des Compactins, eines der ersten Statine, angesehen, allerdings geht die Veröffentlichung davon aus, daß zur Isolierung dieser Verbindung eine Spaltung des Racemats 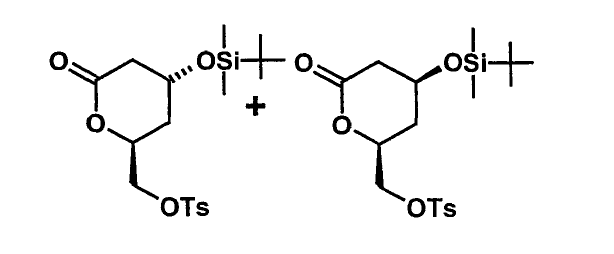 erforderlich ist, was das Gesamtverfahren ineffektiv mache.

Der Stand der Technik ging dann auch zur Herstellung der modernen Statine andere Wege, die nicht über diese silylierte Zwischenverbindung liefen. Auch die Verwendung eines Alkohols, dessen Hydroxylgruppen in 5- und 6-Stellung durch eine verbrückende Schutzgruppe geschützt sind, wie die Ausgangsverbindung der vorstehenden Synthese, wurde nur noch vereinzelt mit mäßigem Erfolg bei der Statinsynthese verwendet, so z.B. in der EP-A 374 922, die die Herstellung der Verbindung 5,6-0-Isoproyliden-3,5,6-trihydroxyhexansäureethylester offenbart. Das Endprodukt dieser Synthese enthielt das gewünschte (3R,5S)-Isomer allerdings nur in einem Verhältnis von 78:22, was für kommerzielle Zwecke nicht befriedigend ist. Eine Umsetzung dieser Verbindung zu einem Lacton erfolgte nicht.

Neure Verfahren zur Verbesserung der Statinsynthese, wie sie beispielsweise in der EP-A 583 171 beschrieben sind, laufen demgegenüber über Zwischenverbindungen, bei denen die Hydroxylschutzgruppen in 3- und 5-Stellung des Trihydroxyhexanats durch eine verbrückende Schutzgruppe geschützt sind oder über Zwischenverbindungen, bei denen auf verbrückende Schutzgruppen vollständig verzichtet wird und die nicht über eine Lactonisierungsreaktion verlaufen.

Typische Beispiele für die Richtung, in die sich der Stand der Technik bei der Statinsynthese bewegt, sind beispielsweise in der WO 03/004450 und der WO 03/004456 beschrieben. Diese Druckschriften offenbaren sogenannte "Schlüsselzwischenprodukte" 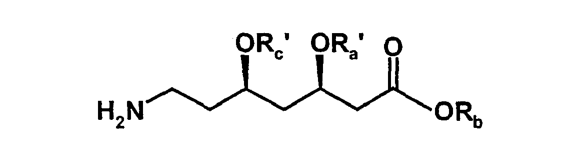 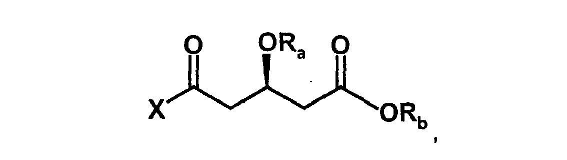 die nach weiteren Umsetzungen mit dem Rest des Statins gekoppelt werden können. Diese "Schlüsselzwischenprodukte" werden durch Verseifung eines racemischen Gemisches der Verbindung 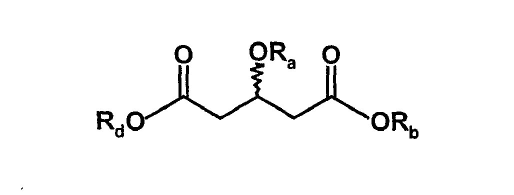 durch einen enantioselektiven Katalysator hergestellt.

Dieses Verfahren bietet den Vorteil, daß die Stereochemie der Statinseitenkette bereits in einem frühen Stadium festgelegt wird, allerdings ist die Verfahrensführung recht komplex, die stereoselektive Hydrolyse der Verbindung 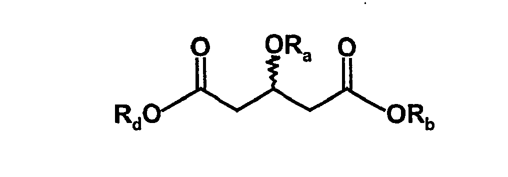 ist mit einem Ausbeuteverlust verbunden, und es besteht das Risiko, daß bei der weiteren Verfahrensführung die Stereochemie der Seitenkette verlorengeht.

Verfahren zur Herstellung von HMG-CoA-Reduktaseinhibitoren oder zur Herstellung der Statinseitenkette sind ebenfalls in Prasad, K. et al., Tetrahedron: Assymetry Vol. 1, Nr. 5, Seiten 307-310, 1990; Prasad, K. et al., Tetrahedron Letters, Vol. 25, Nr. 32, Seiten 3391-3394, 1984; Bennett, F. et al., J. Chem. SOC. Perkins Trans. 1, Seiten 133-140, 1991; DE 35 30 798 und EP-A 1 288 213 beschrieben.

Es besteht ein erheblicher Bedarf nach weiteren Verfahren zur Herstellung von Statinen, die wirtschaftlich sind und mit deren Hilfe die Statine einfach, in hoher Ausbeute und unter Verwendung weniger Verfahrensstufen hergestellt werden können.

Erfindungsgemäß wurde gefunden, daß eine Statinsynthese, die sich an den frühen Versuchen orientiert, wie sie beispielsweise in der Druckschrift J. Org. Chem., Vol. 49, Nr. 21, 1984, 3994-4003 beschrieben sind, dort aber als wenig aussichtsreich angesehen wurden, die Statine mit der gewünschten Statinseitenkette in guter Ausbeute und hoher optischer Reinheit liefert. Einige der Zwischenprodukte sind literaturbekannt, andere der Zwischenprodukte sind neue Verbindungen. Erfindungsgemäß wurde auch ein Verfahren gefunden, mit dem diese Zwischenprodukte einfach und in hoher Ausbeute hergestellt werden können.

Die Erfindung betrifft damit ein Verfahren zur Herstellung eines Statins, umfassend die folgenden Schritte:
a) Herstellung einer Verbindung der Formel II 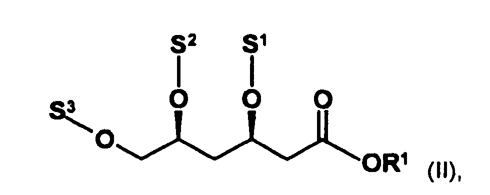 in der
   - S¹: ein Wasserstoffatom oder eine Hydroxylschutzgruppe bedeutet,
   - S² und S³: zusammen eine verbrückende Hydroxylschutzgruppe bedeuten und
   - R¹: ein Wasserstoffatom oder eine Carboxylschutzgruppe darstellt.
   durch stereoselektive Hydrierung einer Verbindung der Formel III 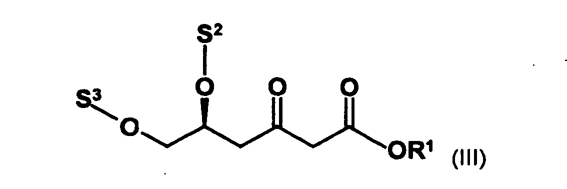 zu einer Verbindung der Formel II-a 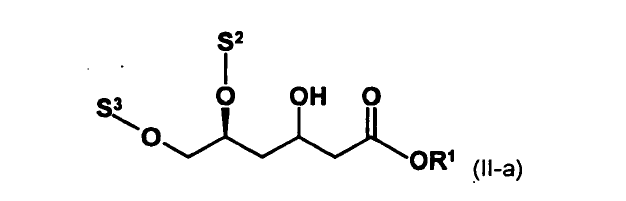 und gegebenenfalls Einführung einer Hydroxylschutzgruppe und
b) Lactonisierung der Verbindung der Formel II zu einer Verbindung der Formel I-a

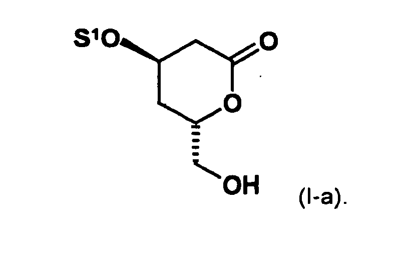

Der Rest S¹ stellt ein Wasserstoffatom oder eine Hydroxylschutzgruppe dar. Hydroxylschutzgruppen sind im Stand der Technik bekannt, und es kann beispielsweise auf die allgemeine Literaturstelle Protective Groups in Organic Synthesis, Theodora W. Greene und Peter G. M. Wuts, 2te Auflage, John Wiley & Sons verwiesen werden. Geeignete Hydroxylschutzgruppen sind auch beispielsweise in der WO 03/004450 genannt, auf die insoweit Bezug genommen wird. Erfindungsgemäß bevorzugt sind Hydroxylschutzgruppen mit 4 bis 10 Kohlenstoffatomen und gegebenenfalls 1 bis 3 Heteroatomen. Besonders bevorzugt enthält die Hydroxylschutzgruppe ein Siliziumatom, 5 bis 10 Kohlenstoffatome und keine weiteren Heteroatome. Insbesondere bevorzugt ist die Hydroxylschutzgruppe S¹ eine Trimethylsilyl-, Triisopropylsilyl-, Triethylsilyl-, tert-Butyldimethylsilyl-, Di-tert-Butylmethylsilyl-, tert-Butyldiphenylsilyl-, Triphenylsilyl-, Diphenylmethylsilyl- oder Tris(trimethylsilyl)schutzgruppe. Am meisten bevorzugt ist die Hydroxylschutzgruppe S¹ eine tert-Butyldimethylsilylgruppe. Bevorzugt sind auch Schutzgruppen der allgemeinen Formel R-O-C(O)- und R-C(O)-, wobei R eine Alkylgruppe, insbesondere eine C₁-C₆-Alkylgruppe, wie eine tert.Butylgruppe, oder eine Arylgruppe, insbesondere eine C₅-C₁₀-Arylgruppe wie eine Phenylgruppe, oder eine Alkyl-Arylgruppe, insbesondere eine C₁-C₆-Alkyl-C₅-C₁₀-Arylgruppe, darstellt.

S² und S³ bilden zusammen eine verbrückende Hydroxylschutzgruppe wie sie prinzipiell bekannt ist. Beispiele von geeigneten verbrückenden Hydroxylschutzgruppen sind in der WO 03/004450 offenbart, auf die insoweit Bezug genommen wird. Besonders bevorzugt bilden die Schutzgruppen S² und S³ zusammen eine Isopropylidenschutzgruppe.

Der Rest R¹ bedeutet ein Wasserstoffatom oder eine Carboxylschutzgruppe. Carboxylschutzgruppen sind dem Fachmann bekannt und beispielsweise in Protective Groups in Organic Synthesis, Theodora W. Greene und Peter G. M. Wuts, 2te Auflage, John Wiley & Sons beschrieben. R¹ kann beispielsweise ein Wasserstoffatom, einen C₁₋₃-Alkyl- oder C₅₋₁₀-Arylrest bedeuten, die gegebenenfalls mit einem oder mehreren Resten substituiert sind, die unabhängig voneinander ausgewählt sind aus Halogenatomen, C₁-C₁₀-Alkylresten, C₅-C₁₀-Alkoxyresten, Heterocyclen, die aus 0 bis 10 Kohlenstoffatomen, bevorzugt 1 bis 5 Kohlenstoffatomen, und 1 bis 10 Heteroatomen, bevorzugt 1 bis 5 Heteroatomen, ausgewählt aus Schwefel, Stickstoff und Sauerstoffatomen, aufgebaut sind, und funktionellen Gruppen. Bevorzugt bedeutet R¹ einen C₁₋₈-Alkyl- oder C₅₋₁₀-Arylrest, die gegebenenfalls mit einem oder mehreren Resten substituiert sind, die unabhängig voneinander ausgewählt sind aus Halogenatomen, Tetrazolyl-, C₁₋₈-Alkyl-, C₁₋₈-Alkoxy-, Nitro- und Cyano-Gruppen.

Der Rest R¹ ist besonders bevorzugt ein C₁₋₈-Alkylrest, insbesondere ein C₁₋₃-Alkylrest, wobei ein Ethylrest am meisten bevorzugt ist, insbesondere wenn der Rest S¹ eine tert-Butyldimethylsilylgruppe darstellt.

Die Verbindung der Formel I-a 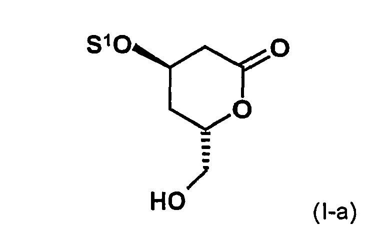 kann auf einfache Art und Weise in Verbindungen der Formel I 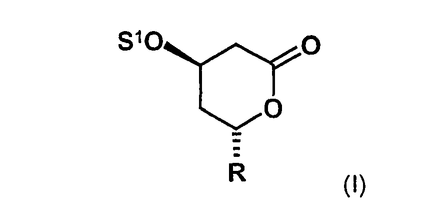 umgesetzt werden, die wichtige Zwischenverbindungen bei der Herstellung von Statinen sind.

In den Verbindungen der Formel I 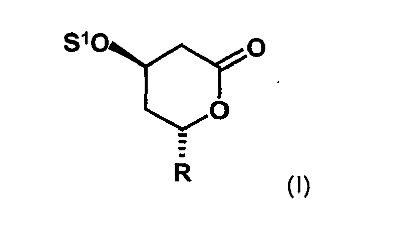 ist der Rest S¹ wie vorstehend definiert. Der Rest R ist ein Rest, über den die Verbindung der Formel I an den Rest des Statins gekoppelt werden kann, insbesondere eine -CH₂R², -CHO, -CH=P(R³)₃, 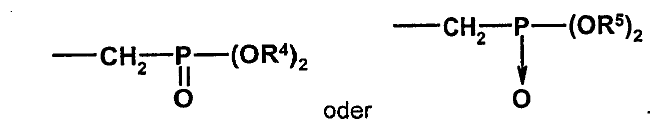 -Gruppe. Die Gruppe -CH=P(R³)₃ liegt im Gleichgewicht mit der Gruppe -⁻CH-⁺P(R³)₃ vor. Hiervon werden daher auch Gruppen -CH₂-P⁺(R³)₃M⁻ umfaßt, wobei M- ein übliches Gegenion darstellt, z.B. Hal⁻ (Hal = Cl, Br oder I) oder ⁻O-Tos. Ist der Rest R eine -CH=P(R³)₃, 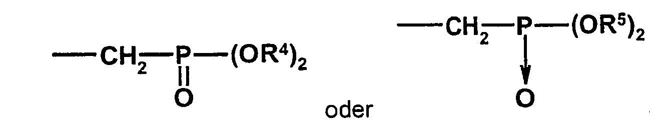 -Gruppe, ist die Verbindung der Formel I ein Wittig-Reagenz oder ein Horner-Wittig-Reagenz, das später mit dem entsprechend funktionalisierten Ringsystem St des Statins eine Wittig-Reaktion bzw. eine Horner-Wittig-Reaktion eingehen kann. Das Ringsystem St, mit dem die Verbindung der Formel 1 zu dem Statin umgesetzt wird, sollte in diesem Fall an der Kopplungsstelle bevorzugt eine Aldehydgruppe tragen.

Die Reste R³, R⁴ und R⁵ sind bevorzugt die üblichen Gruppen, die einen Wittig-Rest oder einen Horner-Wittig-Rest vervollständigen, so daß die Verbindungen eine Wittig-Reaktion bzw. eine Horner-Wittig-Reaktion eingehen können. Der Rest R³ bedeutet damit üblicherweise einen C₅- bis C₁₀-Arylrest, der gegebenenfalls mit ein oder zwei C₁-C₄-Alkylresten und/oder Halogenatomen substituiert ist, einen C₁-C₄-Alkylrest oder einen C₅-C₁₀-Cycloalkylrest, insbesondere einen Phenylrest, einen n-C₁C₄-Alkylrest oder einen Cyclohexylrest. Der Phenylrest ist bevorzugt unsubstituiert. Bevorzugt ist der Phenylrest auch mit ein oder zwei n-C₁-C₄-Alkylresten oder Chloratomen substituiert. Der Rest R⁴ ist bevorzugt ein C₁-C₄-Alkylrest, insbesondere ein n-C₁-C₄-Alkylrest, besonders bevorzugt eine Ethylgruppe, der Rest R⁵ ist bevorzugt ein C₅-C₁₀-Arylrest oder ein C₁-C₆-Alkylrest, insbesondere ein C₅-C₁₀-Arylrest oder eine C₁-C₄-Alkylgruppe, besonders bevorzugt eine Phenyl-, Methyl- oder Ethylgruppe. Diese Reste sind aber nicht besonders eingeschränkt, sofern mit ihnen die später erforderliche Wittig-(bzw. Horner-Wittig)-Reaktion durchführbar ist.

Falls der Rest R in der Verbindung der Formel I eine Aldehydgruppe darstellt, solite das Ringsystem St, mit dem die Verbindung der Formel I zu dem entsprechenden Statin umgesetzt wird, eine entsprechende funktionelle Gruppe aufweisen, so daß eine Wittig-Reaktion oder eine Horner-Wittig-Reaktion durchgeführt werden kann.

Die Wittig-Reaktion und die Horner-Wittig-Reaktion sind bekannte Umsetzungen, und es kann auf einschlägige Lehrbücher der organischen Chemie verwiesen werden, z.B. auf March, Advanced Organic Chemistry, 4te Auflage, 1992, John Wiley and Sons.

Wenn der Rest R einen Rest -CH₂R² darstellt, stellt der Rest R² erfindungsgemäß ein Halogenatom, insbesondere ein Chlor-, Brom- oder lodatom, eine Cyanidgruppe (-C≡N), eine -CH₂NH₂-Gruppe, eine Gruppe SO₂-R⁶ oder eine Abgangsgruppe dar.

Falls R² eine Cyanidgruppe darstellt, ist die Verbindung der Formel I insbesondere ein Zwischenprodukt zur Herstellung einer Verbindung der Formel I, in der R² eine -CH₂NH₂-Gruppe darstellt. Die Verbindung der Formel I, in der R² eine -CH₂NH₂-Gruppe darstellt, ist ein besonders bevorzugtes Zwischenprodukt, das zur Herstellung des Atorvastatins geeignet ist.

Eine Verbindung der Formel I, in der der Rest R² eine Cyanidgruppe darstellt, kann durch Hydrierung in eine Verbindung der Formel I überführt werden, in der R² eine -CH₂NH₂-Gruppe darstellt.

Die Verbindungen der Formel I, in denen der Rest R² einen Rest -SO₂R⁶ bedeutet, können entsprechend den Verbindungen, in denen der Rest R einen Wittig-Rest oder einen Horner-Wittig-Rest bedeutet, mit einem Ringsystem St, das beispielsweise eine Aldehydgruppe als Kopplungsgruppe trägt, zu einem Statin umgesetzt werden. Die entsprechenden Sulphone können entweder direkt von den Alkoholen der Formel I-a oder von den Tosylaten der Formel I erhalten werden, beispielsweise durch Umsetzung mit Sulfiden und anschließender Oxidation mit Peroxiden oder H₂O₂, wie es beispielsweise in Tetrahedron Letters, 1973, 49, 4833-4836; Synlett 1988, 26-28 oder J. Am. Chem. Soc. 2001, 123, 10772-10773 beschrieben ist.

Der Rest R⁶ bedeutet ein Wasserstoffatom, einen C₁₋₃-Alkyl- oder C₅₋₁₀-Arylrest, die gegebenenfalls mit einem oder mehreren Resten substituiert sind, die unabhängig voneinander ausgewählt sind aus Halogenatomen, C₁-C₁₀-Alkylresten, C₁-C₁₀-Alkoxyresten, Heterocyclen, die aus 0 bis 10 Kohlenstoffatomen, bevorzugt 1 bis 5 Kohlenstoffatomen, und 1 bis 10 Heteroatomen, bevorzugt 1 bis 5 Heteroatomen, ausgewählt aus Schwefel, Stickstoff und Sauerstoffatomen, aufgebaut sind, und funktionellen Gruppen. Bevorzugt bedeutet der Rest R⁶ ein Wasserstoffatom, einen C₁₋₈-Alkyl- oder C₅₋₁₀-Arylrest, die gegebenenfalls mit einem oder mehreren Resten substituiert sind, die unabhängig voneinander ausgewählt sind aus Halogenatomen, Tetrazolyl-, C₁₋₈-Alkyl-, C₁₋₈-Alkoxy-, Nitro- und Cyano-Gruppen.

Der Rest R² kann erfindungsgemäß auch eine übliche Abgangsgruppe sein, die im Rahmen einer nucleophilen Substitutionsreaktion eine Kopplung mit einem geeignet substituierten Statinrest ermöglicht. Geeignete Abgangsgruppen sind in der organischen Chemie bekannt, bevorzugt kann hier auf Halogenatome, insbesondere Chlor-, Brom- und lodatome und Reste -O-SO₂-R verwiesen werden, wobei R einen Alkyl-, Aryl- oder Alkylarylrest darstellt, bevorzugt mit nicht mehr als 20 Kohlenstoffatomen, besonders bevorzugt einen C₁-C₆-Alkylrest oder einen C₅-C₁₀-Arylrest, der gegebenenfalls mit 1 oder 2 C₁-C₆-Alkylresten substituiert ist, wie eine Phenylgruppe oder eine p-Tolylgruppe. Besonders bevorzugt ist der Rest -O-SO₂-R eine -O-Tos-Gruppe, wobei Tos für eine Tosylgruppe steht.

Besonders bevorzugt ist der Rest R² eine Cyanidgruppe, eine -CH₂NH₂-Gruppe oder ein Rest SO₂R⁶. Für alle Bedeutungen von R² ist die Hydroxylschutzgruppe S¹ wie vorstehend definiert insbesondere auch in Bezug auf ihre bevorzugten Bedeutungen.

Die Verbindungen der Formel I, in denen der Rest R² ein Halogenatom bedeutet, können bevorzugt direkt aus den Verbindungen der Formel I-a herstellt werden. Verbindungen der Formel I, in denen der Rest R² ein Halogenatom bedeutet, können auch aus Verbindungen der Formel I hergestellt werden, in denen der Rest R² eine andere Abgangsgruppe bedeutet, insbesondere eine O-Tosylgruppe. Die Herstellung von Verbindungen der Formel I, in denen der Rest R² eine -O-Tosylgruppe darstellt, aus den Verbindungen der Formel I-a ist im Stand der Technik bekannt. Die Verbindungen der Formel I, in denen der Rest R² ein Halogenatom bedeutet, können beispielsweise durch Umsetzung mit einer Verbindung P(R⁴)₃ in eine Verbindung der Formel I überführt werden, in der der Rest R eine -CH=P(R³)₃-Gruppe bedeutet.

Die Verbindungen der Formel I-a können z.B. gemäß folgendem Schema zu bevorzugten Verbindungen der Formel I umgesetzt werden: 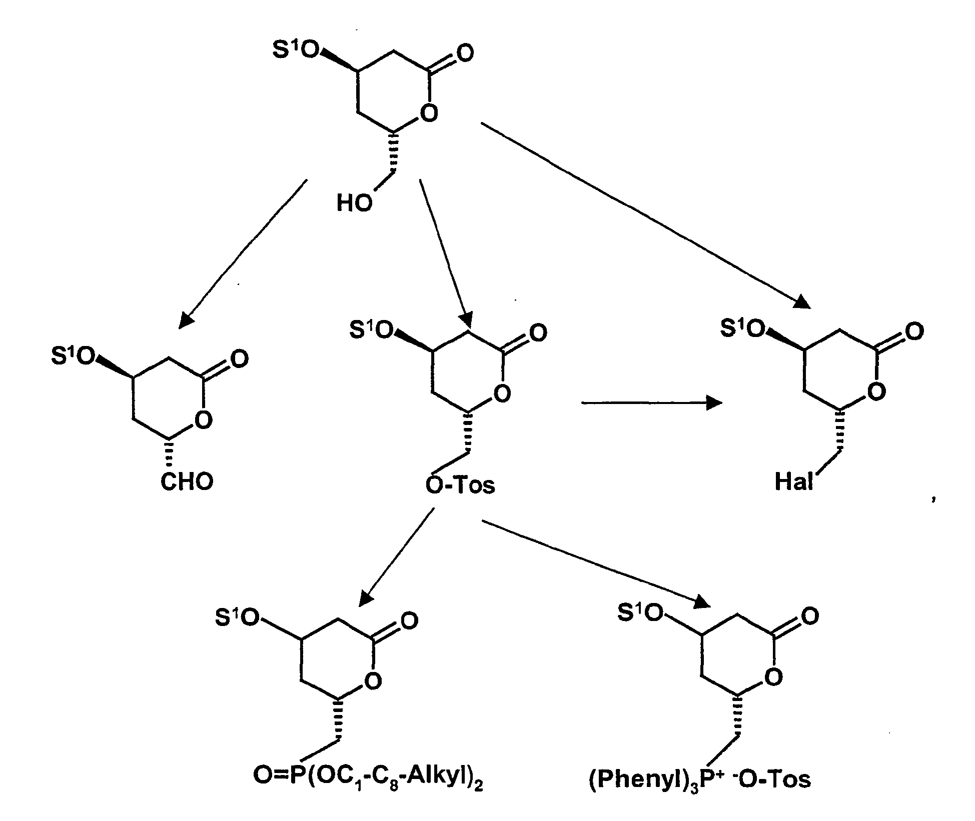 wobei Hal ein Halogenatom bedeutet.

Die Oxidation einer primären OH-Gruppe zu einer Aldehydgruppe kann z.B. über eine Swern-Oxidation erfolgen oder durch Oxidation mit Cr(VI): (PyH)₂Cr₂O₇ - Handbook of Reagents for Organic Synthesis "Oxidizing and Reducing Agents", Ed. S. D. Burke, R. L. Danheiser, John Wiley&Sons Ltd. 1999, S. 330-334 oder mit Cr(VI): HPyCrClO₃ - Handbook of Reagents for Organic Synthesis "Oxidizing and Reducing Agents", Ed. S. D. Burke, R. L. Danheiser, John Wiley&Sons Ltd. 1999, S. 323-330.

Die Überführung der Tosylgruppen in ein Halogenid kann z.B. erfolgen, wie es in Weygand/Hilgetag, 4 Auflage, 1970, S. 232-235 beschrieben ist. Die Überführung der Tosylgruppen in Cyanid ist z.B. im Organikum, 16. Auflage, 1986, 211-216; P. Kurtz in: Houben-Weil, Bd. 8, 1952, S. 290-311; D. T. Mowry, Chem. Rev. 1948, 42, 189-284 beschrieben.

Einzelheiten zu der Herstellung vorstehender Verbindungen können ebenfalls beispielsweise folgenden Druckschriften entnommen werden:
- Journal of the Chemical Society, Perkin Transactions 1: Organic and Bioorganic Chemistry (1972-1999) (1988), (8), 2291-5; (für den Aldehyden)
- Journal of Organic Chemistry (2001), 66 (20), 6803-6806; (für das Tosylat)
- Tetrahedron (1995), 51 (48), 13217-38; (für das Tosylat)
- Journal of the Chemical Society, Perkin Transactions 1: Organic and Bioorganic Chemistry (1995), (13), 1641-3; (für das Tosylat)
- Journal of Organic Chemistry (1984), 49 (21), 3994-4003; (für das Tosylat)
- Fujisawa, Kamotsu et al., JP 10087568 A2, 1998, 0407 Heisei; (für das Chlorid)
- Chemistry Letters (1997), (8), 765-766; (für das Chlorid)
- Tetrahedron Letters (1996), 37 (33), 6001-6004; (für das lodid)
- Journal of the Chemical Society, Perkin Transactions 1: Organic and Bioorganic Chemistry (1972-1999) (1991), (1), 133-40 (für das lodid) und
- Tetrahedron Letters (1988), 29 (38), 4865-8 (für das lodid).

Die meisten Verbindungen der Formel I sind neu. Verbindungen der Formel I, in denen der Rest S¹ eine tert.-Butyldimethylsilylgruppe darstellt und der Rest R eine -CHO, -CH₂-O-Tos, -CH₂Cl oder -CH₂l-Gruppe darstellt, sind bekannt. Besonders bevorzugt sind Verbindungen der Formel I, wobei der Rest S¹ eine tert.-Butyldimethylsilylgruppe darstellt und der Rest R eine -CH₂R², -CH=P(R³)₃, -CH₂-P⁺(R³)₃M⁻, 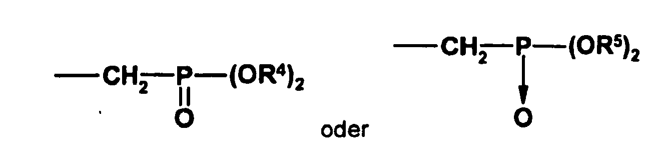 -Gruppe darstellt, wobei R² ein Bromatom, eine C=N, eine -CH₂NH₂-Gruppe oder einen Rest -SO₂-R⁶ darstellt und R³, R⁴, R⁵, R⁶ und M⁻ wie vorstehend definiert sind. Diese Verbindungen können aufgrund der vorstehenden Ausführungen leicht hergestellt werden.

Die WO 93/06235 offenbart in allgemeiner Form bestimmte Tetrahydropyran-2-on-Isomere.

Bevorzugte Verbindungen der Formel I 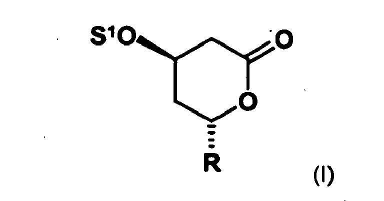 sind diejenigen Verbindungen,
in denen
- S¹: wie vorstehend definiert ist,
- R: -CH₂R², -CHO oder -CH=P(R³)₃ bedeutet,
- R²: -CH₂NH₂, -SO₂-R⁶ oder einen
Rest -O-SO₂R⁷ bedeutet, wobei R⁷ einen Alkyl-, Aryl- oder Alkylarylrest darstellt,
mit der Maßgabe, daß der Rest S¹ keine tert.-Butyldimethylsilylgruppe darstellt, wenn der Rest R eine CHO-Gruppe oder CH₂-OTos-Gruppe darstellt,
- R³: einen Wittig-Rest oder einen Horner-Wittig-Rest vervollständigt,
- R⁶: ein Wasserstoffatom, einen C₁₋₃-Alkyl- oder C₅-₁₀-Arylrest bedeutet, die gegebenenfalls mit einem oder mehreren Resten substituiert sind, die unabhängig voneinander ausgewählt sind aus Halogenatomen, Heterocyclen, die 0 bis 10 Kohlenstoffatome und 1 bis 10 Heteroatome, ausgewählt aus Schwefel, Stickstoff und Sauerstoffatomen, enthalten und funktionellen Gruppen und
- M⁻: ein Gegenion darstellt.

Noch stärker bevorzugt sind diejenigen Verbindungen, in denen der Rest S¹ eine tert.-Butyldimethylsilylgruppe darstellt und der Rest R eine -CH₂R² oder -CH=P(R³)₃-Gruppe darstellt, wobei R² eine -CH₂NH₂-Gruppe oder einen Rest -SO₂-R⁶ darstellt,
- R³: einen Wittig-Rest oder einen Horner-Wittig-Rest vervollständigt,
- R⁶: ein Wasserstoffatom, einen C₁₋₃-Alkyl- oder C₅₋₁₀-Arylrest bedeutet, die gegebenenfalls mit einem oder mehreren Resten substituiert sind, die unabhängig voneinander ausgewählt sind aus Halogenatomen, Heterocyclen, die 0 bis 10 Kohlenstoffatome und 1 bis 10 Heteroatome, ausgewählt aus Schwefel, Stickstoff und Sauerstoffatomen, enthalten und funktionellen Gruppen und
- M⁻: ein Gegenion darstellt.

Es wird ebenfalls auf die nachstehenden Beispiele verwiesen.

Erfindungsgemäß werden die Verbindungen der Formel I-a 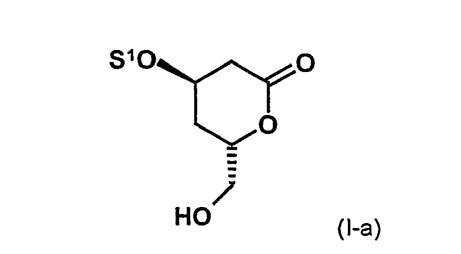 ausgehend von Verbindung II 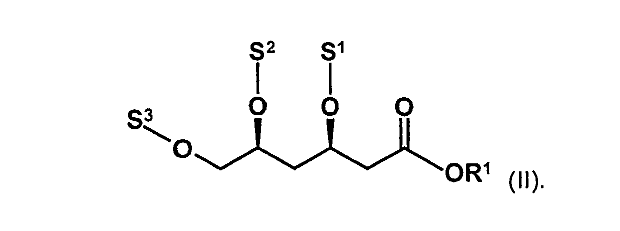 wobei die Reste S¹, S², S³ und R¹ wie vorstehend definiert sind, hergestellt.

Dieser Verfahrensschritt ist im Prinzip literaturbekannt und kann beispielsweise durch Erhitzen in Essigsäure erfolgen. Es wird beispielsweise auf die folgenden Druckschriften verwiesen:
- Journal of Organic Chemistry (1984), 49 (21), 3994-4003;
- EP-A 1 234 885;
- US-B 6,417,374;
- Journal of Organic Chemistry (2001), 66 (20), 6803-6806;
- Tetrahedron (1995), 51 (48), 13217-38;
- Journal of the Chemical Society, Perkin Transactions 1: Organic and Bioorganic Chemistry (1995), (13), 1641-3,
auf die insoweit vollständig Bezug genommen wird.

Die Verbindung der Formel II 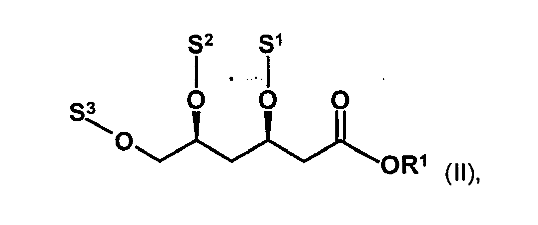 in der S¹ eine Hydroxylschutzgruppe darstellt, kann leicht aus Verbindungen der Formel II, in der der Rest S¹ ein Wasserstoffatom darstellt, d.h. Verbindungen der Formel II-a, hergestellt werden, z.B. durch Umsetzung mit einer Verbindung S¹-A, wobei A eine übliche Abgangsgruppe wie ein Halogenatom darstellt (z.B. Chlor, Brom oder lod) und S¹ die Hydroxylschutzgruppe darstellt.

Die besonders bevorzugte Verbindung der Formel II, in der die Schutzgruppe S¹ eine t-Butyldimethylsilylschutzgruppe darstellt, kann z.B. vorteilhaft durch Umsetzung der Verbindung II-a mit CISiMe₂tert-Butyl in einem Gemisch aus DMF und Imidazol erfolgen.

Die Verbindung der Formel II-a 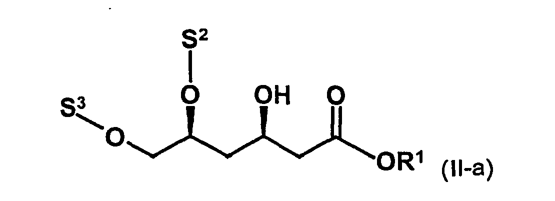 kann mit hoher Stereoselektivität aus der Verbindung der Formel III 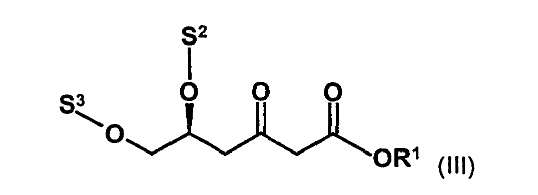 hergestellt werden, z.B. durch Hydrierung mit Wasserstoff bei Raumtemperatur (25°C) unter erhöhtem Druck im Bereich von 20 bis 80 bar, insbesondere 30 bis 70 bar, z.B. etwa 50 bar in einem geeigneten Lösemittel, bevorzugt einem polar protischen Lösungsmittel, insbesondere in einem C₁- bis C₆-Alkohol, wie Methanol oder Ethanol. Die Hydrierung erfolgt bevorzugt mit einem sogenannten Ru-BINAP-Katalysator, wie er beispielsweise im Tetrahedron Lett. 1991, 32, 4163 und in der WO 95/18784 beschrieben ist, und auf diese beiden Druckschriften wird für die Definition und die Herstellung des bevorzugten Katalysators zur Durchführung des erfindungsgemäßen Verfahrens vollinhaltlich Bezug genommen.

Die erfindungsgemäß am meisten bevorzugten Katalysatoren haben die Struktur

(R)-Ru(BINAP)Cl₂ x NEt₃ (Et = Ethyl) oder

(R)-Ru(BINAP)Cl₂ x DMF x NEt₃,

wobei BINAP die Formel 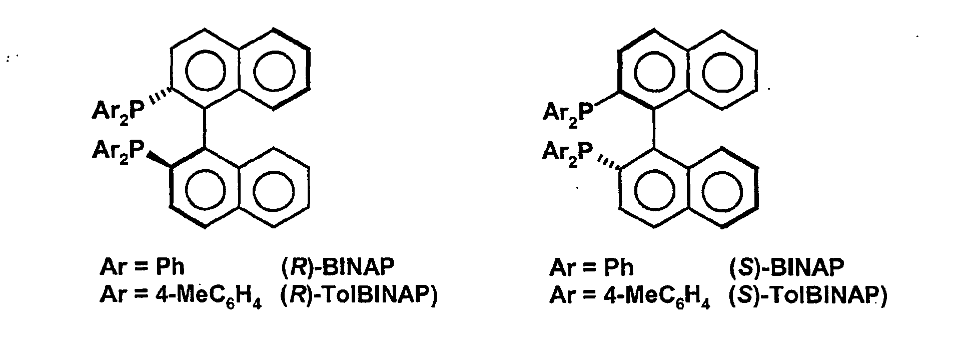 (Ph = Phenyl) aufweist und hergestellt werden kann, wie es z.B. in Tetrahedron Lett. 1991, 32, 4163 beschrieben ist. Anstelle von (R)-BINAP kann vorteilhaft ebenfalls (R)-Tol BINAP verwendet werden. Mit dem Katalysator (R)-Ru(BINAP)Cl₂ x NEt₃ kann bei der erfindungsgemäßen Umsetzung ein syn/anti-Verhältnis von ≥ 80, insbesondere von ≥ 90, bevorzugt ≥ 95, stärker bevorzugt ≥ 99, erreicht werden, was einem molaren Verhältnis des gewünschten (3R,5S)-Isomers zu dem unerwünschten Isomer von 80:20 oder besser entspricht. Demgegenüber konnte im Stand der Technik, wie er z.B. in der EP-A 374 922 beschrieben ist, nur ein molares Verhältnis von 78:22 erreicht werden. Auch mit dem entsprechenden (S)-BINAP Katalysator erhält man eine stereoselektive Umsetzung, allerdings überwiegt das ungünstige anti-Isomer. Die Erfindung betrifft damit auch ein Verfahren zur stereoselektiven Hydrierung einer Verbindung der Formel III zu einer Verbindung der Formel II-a, insbesondere unter Verwendung eines (R)-RuBINAP- oder (R)-RuTolBINAP-Katalysators, wobei diese Katalysatoren wie vorstehend definiert sind und z.B. in der WO 95/18784 oder in Tetrahedron Lett. 1991, 32, 4163 beschrieben werden, wobei die Hydrierung ein molares Verhältnis zwischen der Verbindung der Formel II-a 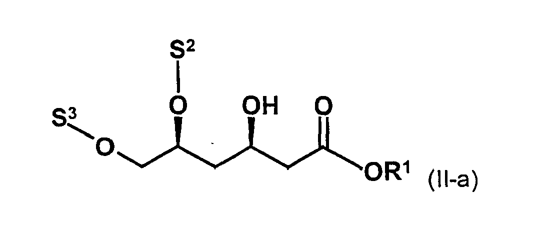 und der entsprechenden anti-Verbindung 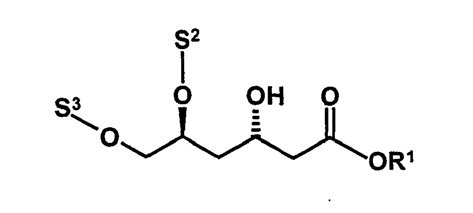 von 80:20 oder mehr, insbesondere von 90:10 oder mehr, insbesondere von 95:5 oder mehr und am stärksten bevorzugt von 99:1 oder mehr liefert. In der Regel ist es daher bei dem erfindungsgemäßen Verfahren nicht mehr erforderlich, das unerwünschte anti-Isomer abzutrennen. Falls dies dennoch notwendig sein sollte, kann es auf bekannte Art und Weise erfolgen.

Die Verbindung der Formel III ist leicht z.B. entsprechend dem Verfahren von Angew. Chem. 1979, 91, 76-77 aus der Verbindung der Formel IV 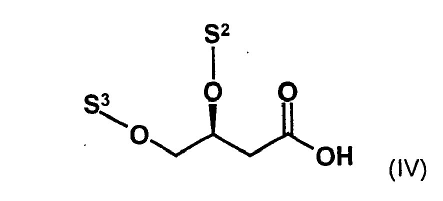 erhältlich, die z.B. auf bekannte Art und Weise aus der kommerziell erhältlichen und preiswerten S-Äpfelsäure erhalten werden kann.

Die Umsetzung der Verbindung IV zu der Verbindung III erfolgt vorteilhafterweise indem zunächst die Carboxylgruppe der Verbindung IV mit einem geeigneten Aktivierungsmittel wie N,N'-Carbonyldümidazol aktiviert wird. Die aktivierte Verbindung wird anschließend mit einer Verbindung M¹R⁷₂X₀₋₁ umgesetzt wird. Hierbei ist M¹ ein zwei- oder dreiwertiges Metallkation, insbesondere ein Metallkation der zweiten oder dritten Hauptgruppe oder der zweiten oder dritten Nebengruppe des Periodensystems der Elemente, insbesondere ein Magnesium-, Calcium-, Zink- oder Aluminiumion, besonders bevorzugt ein Magnesium-, Zink-, oder Aluminiumion (Mg²⁺, Zn²⁺ oder Al³¹⁺-Ionen), und der Rest R⁷ ist ein geeigneter Carbonsäurerest, insbesondere ein partiell veresterter Dicarbonsäurerest wie beispielsweise ein C₁₋₄-O₂C(CH₂)-₄CO₂-Rest, z.B. ein EtO₂CCH₂CO₂-Rest. Ein weiteres Beispiel für einen geeigneten Rest R⁷ ist ein C₁₋₆COO-Rest wie ein CH₃COO-Rest. Die beiden Reste R⁷ an dem Metallkation können identisch sein, es können aber auch zwei verschiedene Reste R⁷ an dem Metallion vorhanden sein. Der Rest X stellt ein gegebenenfalls vorhandenes einwertiges Gegenion dar, das zum Ladungsausgleich dient falls es sich bei dem Metallkation M¹ um ein dreiwertiges Ion handelt. Besonders bevorzugt sind die beiden Reste R⁷ unterschiedlich, beispielsweise ist einer der Reste R⁷ ein C₁₋₄-O₂C(CH₂)₁₋₄CO₂-Rest und der andere Rest ein C₁-C₆COO-Rest. Sehr gute Ergebnisse können beispielsweise mit den Verbindungen Mg(CH₃COO⁻)(EtO₂CCH₂CO2⁻), Zn(EtO₂CCH₂CO₂⁻)(EtO₂CCH₂COO⁻) oder AlCl(EtO₂CCH₂CO2⁻)(EtO₂CCH₂COO⁻) erzielt werden. Andere Kombinationen der vorstehenden Reste sind selbstverständlich erfindungsgemäß ebenfalls möglich. Die Umsetzung kann beispielsweise bei Raumtemperatur in THF erfolgen, und es konnten Ausbeuten von über 60%, vorzugsweise von über 70% erreicht werden.

Die Metallsalze können bevorzugt in situ beispielsweise durch Umsetzen des entsprechenden Metallpulvers mit der entsprechenden Säure (z.B. EtO₂CCH₂COOH unter Rückfluß in einem geeigneten Lösemittel wie einem Ether, z.B. THF, hergestellt werden.

Mit dem erfindungsgemäßen Verfahren können bevorzugt alle Statine hergestellt werden, die die Seitenkette 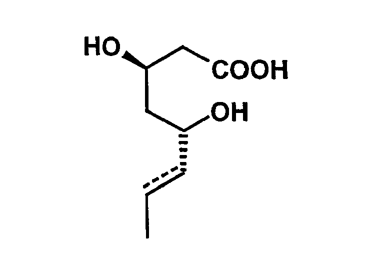 wobei die gestrichelte Linie eine gegebenenfalls vorhandene Bindung darstellt, oder das entsprechende Lacton aufweisen.

Bevorzugt können die folgenden Statine genannt werden: 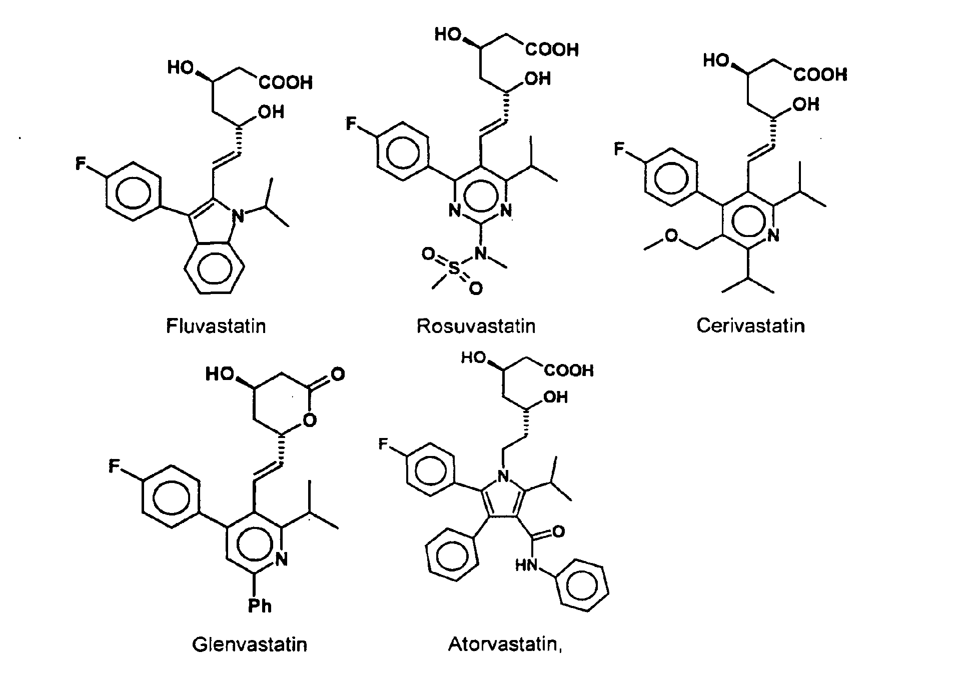 aber auch z.B. Itavastatin und BMY22089.

Statine, die eine Seitengruppe 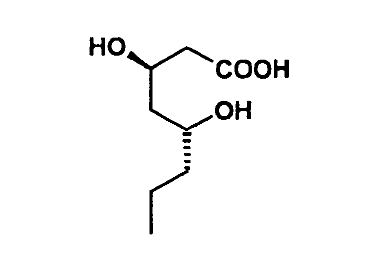 aufweisen, können durch Hydrierung der entsprechenden Statine erhalten werden, die die Seitengruppe 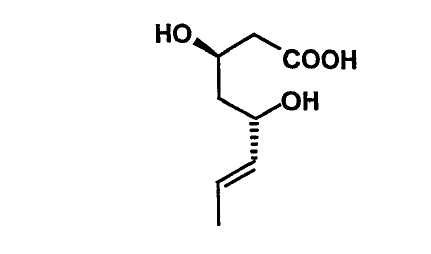 aufweisen. Bevorzugt findet die Hydrierung an einer Vorstufe des Statins statt, bei der die Hydroxylgruppe geschützt ist, d.h. an einem Statin mit der Seitenkette 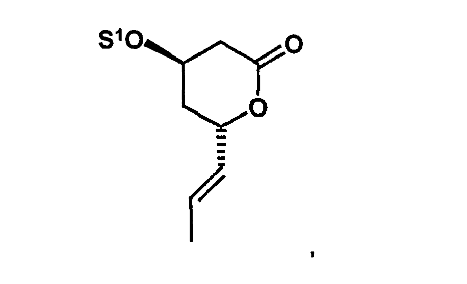 wobei S¹ eine Hydroxylschutzgruppe darstellt und wie vorstehend definiert ist.

Bevorzugt findet die Abspaltung der Schutzgruppe S¹ (falls vorhanden) und die Öffnung des Lactonrings durch Hydrolyse als letzter Schritt der erfindungsgemäßen Statinsynthese statt.

Erfindungsgemäß bevorzugt findet die Kopplung der Verbindung der Formel I mit dem Ringsystem St, das für den Rest des Statins steht, durch eine Wittig-Reaktion oder durch eine Horner-Wittig-Reaktion statt. Hierbei ist der Rest St des Statins entweder mit einer Aldehydgruppe funktionalisiert, insbesondere wenn die Verbindung der Formel I eine Wittig-oder Horner-Wittig-Funktionalität trägt oder das Ringsystem des Rests St ist mit einer Wittig- oder Horner-Wittig-Funktionalität versehen, falls die Verbindung der Formel I eine Aldehydgruppe trägt. Verfahren zur Herstellung entsprechend funktionalisierter Ringsysteme St sind z.B. in den Druckschriften WO 84/02131, EP-A 244 364 und EP 521 471 beschrieben, auf die insoweit vollinhaltlich Bezug genommen wird. Funktionalisierte Ringsysteme St, die in diesen Druckschriften nicht ausdrücklich genannt sind, können auf entsprechende Art und Weise hergestellt werden. Hier kann beispielsweise auf die WO 01/04100 und die WO 03/006439 verwiesen werden.

Ein allgemeines Verfahrensschema zur Herstellung der Statine ist wie folgt 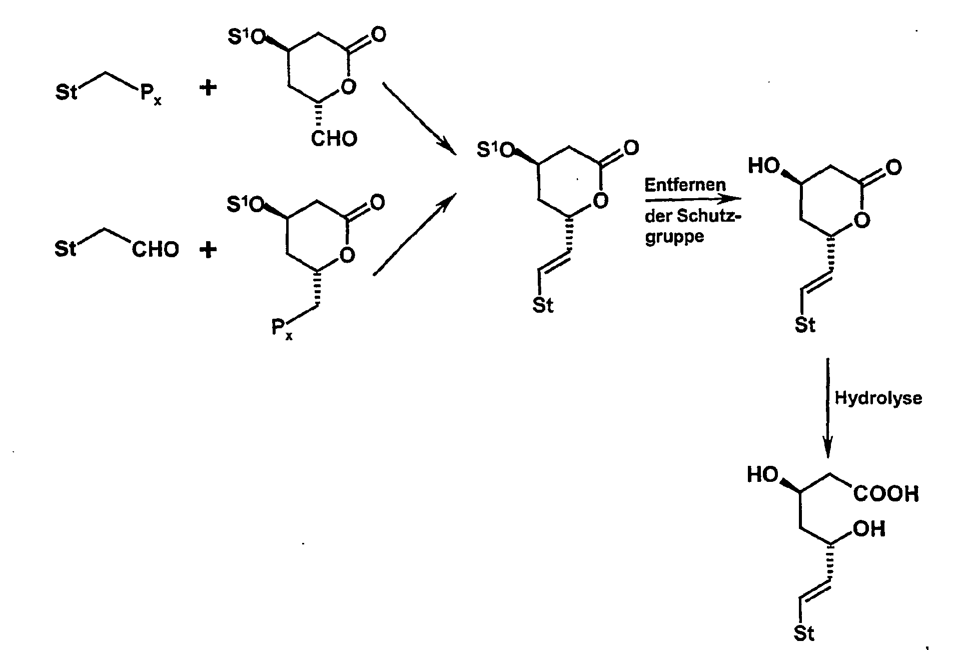 wobei S¹ wie vorstehend definiert ist und Pₓ eine Wittig-Gruppe oder eine Horner-Wittig-Gruppe darstellt, wie vorstehend definiert, insbesondere eine -P⁺(Phenyl)₃⁻OTos-Gruppe, und St den Rest des Statins darstellt, z.B. 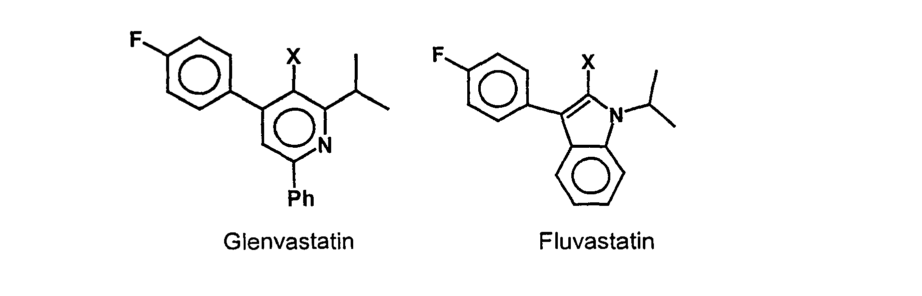 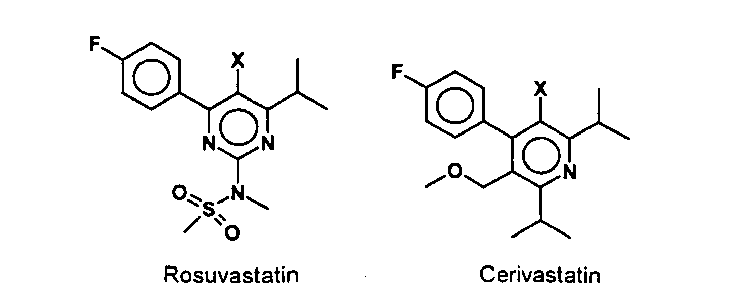 (X zeigt jeweils Bindungsstellen).

In vorstehendem Schema kann der Rest Pₓ auch einen Rest -S(O)₂-R⁶ darstellen, wobei R⁶ wie vorstehend definiert ist.

Ein beispielhaftes Syntheseschema zur Herstellung von Atorvastatin ist wie folgt

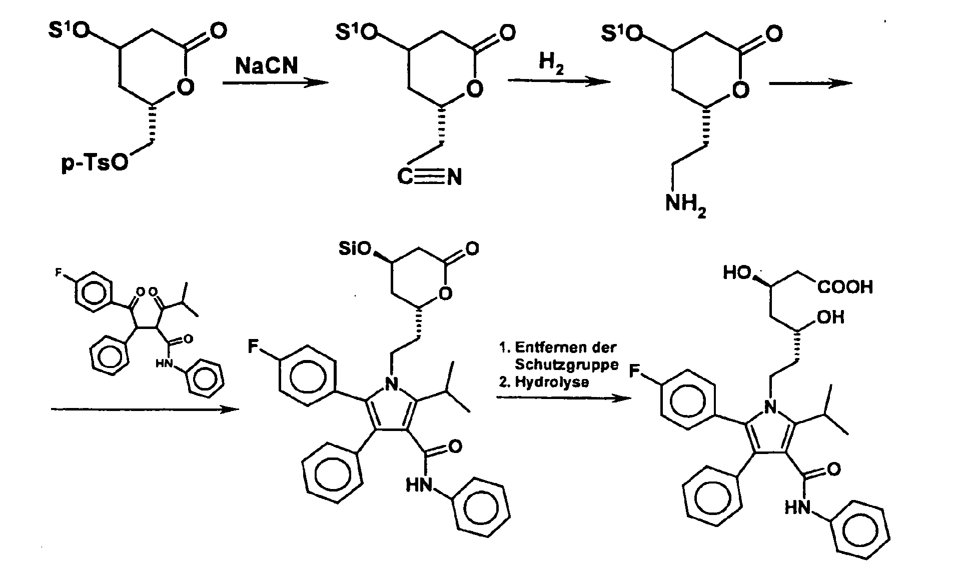

Das Diketon 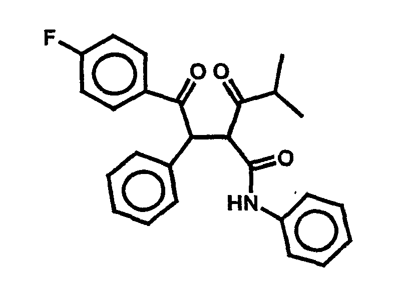 kann beispielsweise hergestellt werden, wie es in WO-A 03/344011, WO-A 03/24959, WO-A 03/04457, WO-A 03/04450, WO-A 01/72706, WO-A 98/04543, US-A 5,298,627, WO 89/07598 oder in Tetrahedron Letters (1992), 33 (17), 2283-4 beschrieben wird.

Die folgenden Beispiele erläutern die Erfindung.

### Beispiele

Die folgenden Beispiele beziehen sich auf das folgende beispielhaft angegebene Syntheseschema:

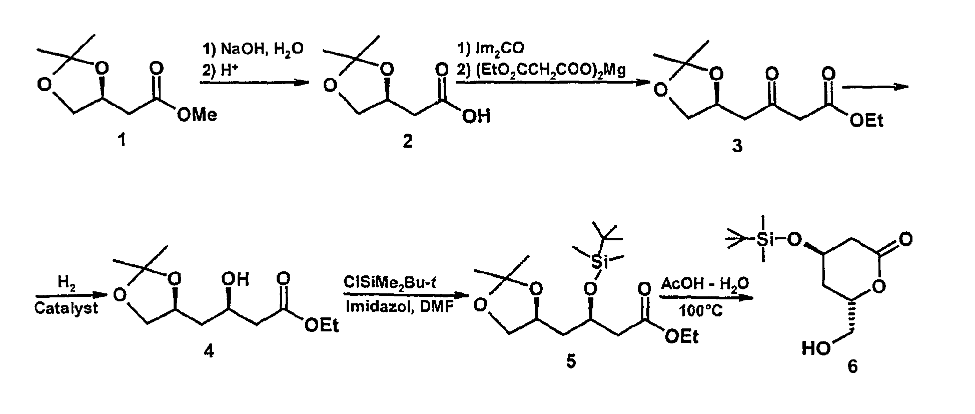

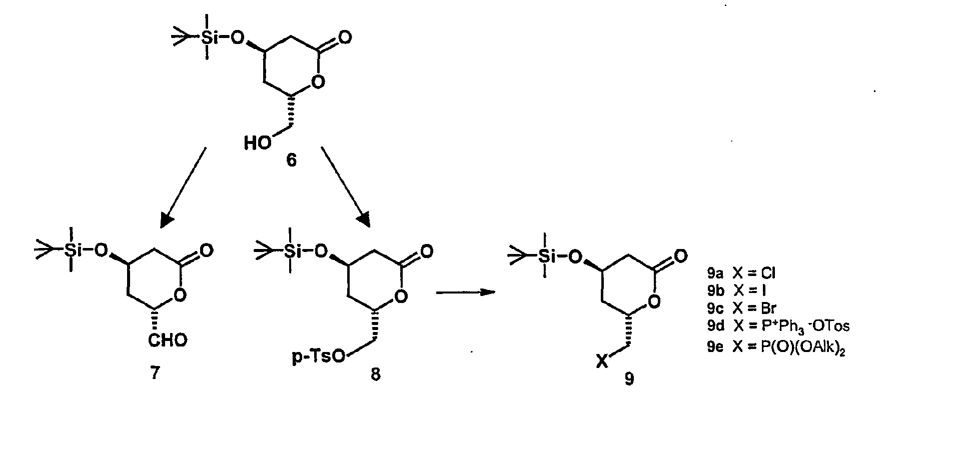

### Beispiel 1

### (4S)-(2,2-Dimethyl-1,3-dioxolan-4-yl)essigsäuremethylester (Verbindung 1)

Diese Verbindung ist kommerziell erhältlich, beispielsweise von der Firma Aldrich, oder sie kann ausgehend von (S)-Äpfelsäuredimethylester auf einfache Art und Weise hergestellt werden, wobei selektiv eine der Estergruppen entsprechend Chem. Letters 1984, 1389-1392 bzw. Tetrahedron 1992, 48, 4067-4086 reduziert wird.

Zu einer Lösung von 113,4 g (0,70 mol) von (S)-Äpfelsäuredimethylester in 300 ml absolutem THF wurden 0,28 g (0,0074 mol) NaBH₄ auf einmal zugegeben. Anschließend wurden langsam 68 ml (54,5 g, 0,72 mol) des BH₃ x Me₂S-Komplexes bei Raumtemperatur unter Rühren zugegeben. Während der Zugabe entwickelten sich gasförmige Produkte. Nach Beendigung der Zugabe wurde das Umsetzungsgemisch 3 Stunden bei Raumtemperatur gehalten. Anschließend wurden 285 ml Methanol zugegeben, und die erhaltene Lösung wurde über Nacht bei Raumtemperatur stehengelassen. Die flüchtigen Bestandteile wurden abgedampft, und der viskose Rückstand wurde 6 Stunden unter vermindertem Druck getrocknet. Der Rückstand wurde mit 300 ml Aceton, 96,3 ml (81,6 g, 0,78 mol) Me₂C(OMe)₂ und 4 g (0,021 mol) p-TsOH x H₂O gemischt. Die Umsetzung wurde über Nacht bei Raumtemperatur gerührt. Anschließend wurde mit 4 g Natriumcarbonat neutralisiert. Das Umsetzungsgemisch wurde 1 weitere Stunde gerührt, filtriert und abgedampft. Der Rückstand wurde unter vermindertem Druck (74°C/6 mbar) destilliert, und man erhielt 90,6 g (74,4%) der Verbindung 1.

### Beispiel 2

### (4S)-(2,2-dimethyl-1,3-dioxolan-4-yl)essigsäure (Verbindung 2)

50 g (0,287 mol) der Verbindung des Beispiels 1 wurden unter Rühren zu einer mit Eis gekühlten 2-molaren wäßrigen Natriumhydroxydlösung (287 ml, 0,574 mol) zugegeben. Das Eisbad wurde entfernt, und das Gemisch wurde 2 Stunden gerührt. Das Gemisch wurde mit Dichlormethan extrahiert (3 x 50 ml), und die organischen Extrakte wurden abgetrennt. Die wäßrige Schicht wurde mit 100 ml Diethylether gemischt und mit Eis gekühlt. Zu dem Gemisch wurden 300 ml 2-normaler wäßriger Natriumhydrogensulfatlösung zugegeben. Das Gemisch wurde 15 Minuten heftig gerührt. Die organische Phase wurde abgetrennt und die wäßrige Phase wurde mit Ethylacetat (2 x 100 ml) extrahiert. Die vereinigten organischen Phasen wurden über Natriumsulfat getrocknet und abgedampft. Der Rückstand wurde unter vermindertem Druck getrocknet, und man erhielt 36 g (78,3% eines flüssigen Produktes. Die mit NMR-Spektrum bestimmte Reinheit des Endproduktes war 95%. Das Produkt konnte ohne weitere Reinigung verwendet werden.

¹H NMR (CDCl₃), δ in ppm: 1,37 (3H, s), 1,43 (3H, s), 2,58 (1H, dd, J = 16,2 und 6,7 Hz), 2,75 (1H, dd, J = 16,2 und 6,7 Hz), 3,68 (1H, dd, J = 8,5 und 6,1 Hz), 4,17 (dd, J = 8,5 und 6,0 Hz), 4,45-4,53 (1 H, m), 11 (1 H, br. s).
¹³C NMR (CDCl₃), δ in ppm: 25,8 (CH₃), 27,2, (CH₃), 39,2 (CH₂), 69,4 (CH₂), 72,1 (CH), 109,9 (C), 176,7 (COO).

### Beispiel 3

### (4S)-(2,2-Dimethyl-1,3-dioxolan-4-yl)-3-oxohexansäureethylester (Verbindung 3)

Ein Gemisch aus 49,0 g (0,371 mol) Malonsäuremonoethylester und 6,0 g (0,248 g-Atom) Magnesium wurden in 200 ml absolutem THF vier Stunden unter Rühren zum Rückfluß erhitzt, wodurch eine erste Lösung erhalten wurde. Parallel hierzu wurde zu einer Lösung von 25,8 g (0,161 mol) der Verbindung des Beispiels 2 in 100 ml absolutem THF 28,8 g (0,177 mol) während 5 bis 10 Minuten festes N,N'-Carbonylbisimidazol zugegeben, wobei eine Gasentwicklung auftrat. Anschließend wurde 2 Stunden bei Raumtemperatur gerührt. Zu dieser Lösung wurde die erkaltete erste Lösung zugegeben. Das verbleibende Magnesium wurde mit 50 ml absolutem THF gewaschen, und die Waschlösung wurde dem Reaktionsgemisch zugegeben. Das Reaktionsgemisch wurde über Nacht bei Raumtemperatur gerührt. Das Reaktionsgemisch wurde abgedampft, der Rückstand wurde in 200 ml Ethylacetat aufgelöst und unter heftigem Rühren mit 430 ml einer 2-normalen wäßrigen Natriumhydrogensulfatlösung angesäuert. Die organische Phase wurde abgetrennt, nacheinander mit 2-normaler wäßriger Natriumhydrogensulfatlösung (2 x 200 ml) und gesättigter wäßriger Natriumhydrogencarbonatlösung (3 x 200 ml) gewaschen, über Natriumsulfat getrocknet und abgedampft. Der Rückstand wurde unter vermindertem Druck (90 bis 93°C, 0,07 mbar) destilliert, und man erhielt 26,9 g (72,4%) der Verbindung 3. Ausweislich der NMR-Spektren in CDCl₃ enthielt das Produkt etwa 10% der Enolform. Das folgende NMR-Spektrum bezieht sich ausschließlich auf die Ketoform.

¹H NMR (CDCl₃), δ in ppm: 1,28 (3H, t, J = 7,1 Hz), 1,35 (1H, s), 1,40 (1H, s), 2,75 (1H, dd, J = 17,1 und 6,8 Hz), 2,99 (1H, dd, J = 17,1 und 6,1 Hz), 3,49 (2H, s), 3,57 (1H, dd, J = 8,4 und 6,6 Hz), 4,15-4,24 (3H, m), 4,42-4,52 (1H, m).
¹³C NMR (CDCl₃), δ in ppm: 14,3 (CH₃), 25,7 (CH₃), 27,1 (CH₃), 47,4 (CH₂), 49,9 (CH₂), 61,7 (CH₂), 69,5 (CH₂), 71,7 (CH), 109,2 (C), 167,1 (COO), 201 (C=O).

### Beispiel 4

### (3R,4S)-(2,2-Dimethyl-1,3-dioxolan-4-yl)-3-hydroxyhexansäureethylester (Verbindung 4)

### a) Herstellung des Katalysators

Ein Gemisch von 200 mg (0,295 mmol) (R)-TolBINAP, 73,6 mg (0,147 mmol) [Ru(C₆H₆)Cl₂]₂ und 2 ml DMF wurde bei 100°C 15 Minuten unter Argon gerührt. Die flüchtigen Bestandteile wurden abgedampft, und der Rückstand wurde unter vermindertem Druck 1 Stunde bei 50°C getrocknet. Dieses Verfahren zur Herstellung von BINAP-Katalysatoren beruht auf der Veröffentlichung in Tetrahedron Lett. 1991, 32, 4163. Der Rückstand wurde in 3 ml Dichlormethan gelöst, und 0,2 ml Triethylamin wurden zugesetzt. Nach 1 Stunde bei Raumtemperatur wurden die flüchtigen Bestandteile abgedampft, und der Rückstand wurde unter vermindertem Druck getrocknet. Das feste Produkt wurde ohne weitere Reinigung und Charakterisierung als Katalysator für die folgende Hydrierung verwendet.

### b) Hydrierung des Ketons des Beispiels 3

Ein Gemisch von 0,58 g (2,5 mmol) der Verbindung des Beispiels 3, 4,3 mg (ca. 0,005 mmol) des in Schritt a) hergestellten Präkatalysators in 10 ml absolutem sauerstofffreien Methanol wurde unter Initial 50 bar Wasserstoffdruck bei Raumtemperatur unter Rühren unter anaeroben Bedingungen hydriert. Nach 150 Minuten war die Wasserstoffaufnahme beendet. Der Autoclav wurde geöffnet, und das Gemisch wurde abgedampft und unter vermindertem Druck getrocknet. Die Umsetzung und die Ausbeute waren quantitativ. Nach den NMR-Spektren war die diastereomere Reinheit des Produkts größer als 99%. Die diastereomere Reinheit wurde anhand der NMR-Spektren durch Analogie zu den entsprechenden Methylestern gemäß Chem. Ber. 1998, 2035-2044 bestimmt.

¹H NMR (CDCl₃), δ in ppm: 1,27 (3H, t, J = 7,1 Hz), 1,36 (3H, s), 1,42 (3H, s), 1,72-1,83 (2H, m), 2,45-2,59 (2H, m), 3,53-3,61 (2H, m), 4,08-4,35 (5H, m).
¹³C NMR (CDCl₃), δ in ppm: 14,4 (CH₃), 25,9 (CH₃), 27,1 (CH₃), 39,9 (CH₂), 41,8 (CH₂), 60,8 (CH₂), 67,0 (CH), 69,7 (CH₂), 74,6 (CH), 109,4 (C), 172,3 (COO).

### Beispiel 5

### (3R,4S)-(2,2-Dimethyl-1,3-dioxolan-4-yl)-3-(tert.-butyldimethylsilyloxy)hexanoat (Verbindung 5)

Die Titelverbindung wurde mit einer Ausbeute von 88% aus der Verbindung des Beispiels 4 nach der Vorschrift in J. Org. Chem. 1984, 49, 3994-4003 erhalten.

### Beispiel 6

### (4R,6S)-4-(tert.-Butyldimethylsilyloxy)-6-hydroxymethyltetrahydropyran-2-on (Verbindung 6)

Die Titelverbindung wurde mit einer Ausbeute von 60% aus der Verbindung des Beispiels 5 nach dem Verfahren in J. Org. Chem. 1984, 49, 3994-4003 erhalten.

### Beispiel 7

### (4R,6S)-4-(tert-Butyldimethylsilyloxy-6-(p-toluolsufonyloxymethyl)tetrahydropyran-2-on (Verbindung 8)

Die Titelverbindung wurde mit einer Ausbeute von 91 % aus der Verbindung des Beispiels 6 entsprechend dem Verfahren nach J. Org. Chem. 1984, 49, 3994-4003 erhalten.

## Patentansprüche

1. Verfahren zur Herstellung eines Statins, umfassend die folgenden Schritte:
a) Herstellung einer Verbindung der Formel II 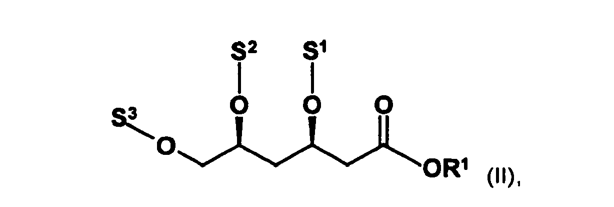 in der
S¹ ein Wasserstoffatom oder eine Hydroxylschutzgruppe bedeutet,
S² und S³ unabhängig voneinander Hydroxylschutzgruppen bedeuten. wobei die Schutzgruppen S² und S³ verbrückt sind, und
R¹ ein Wasserstoffatom oder eine Carboxylschutzgruppe darstellt.
durch stereoselektive Hydrierung einer Verbindung der Formel III 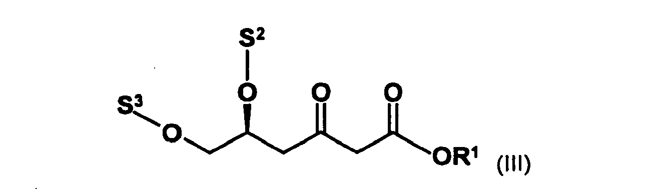 zu einer Verbindung der Formel II-a 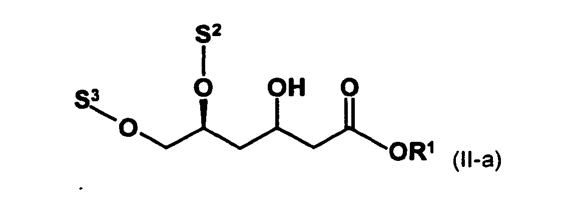 und gegebenenfalls Einführung einer Hydroxylschutzgruppe und
b) Lactonisierung der Verbindung der Formel II zu einer Verbindung der Formel I-a

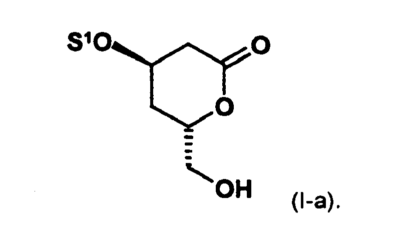

2. Verfahren nach Anspruch 1, umfassend den weiteren Schritt
c) Überführung der Verbindung der Formel I-a 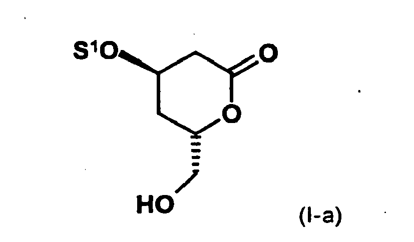 in eine Verbindung der Formel I 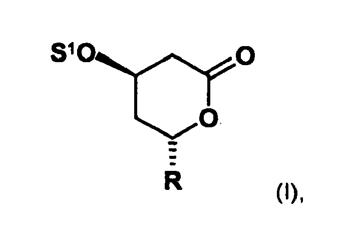 wobei der Rest
S¹ wie in Anspruch 1 definiert ist,
R -CH₂R², -CHO, -CH=P(R³)₃, -CH₂-P⁺(R³)₃M⁻, 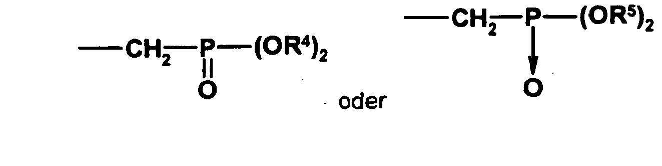 bedeutet,
R² ein Halogenatom, -CΞN, -CH₂NH₂, -SO₂-R⁶ oder eine Abgangsgruppe bedeutet,
R³, R⁴ und R⁵ einen Wittig-Rest oder einen Horner-Wittig-Rest vervollständigen,
R⁶ ein Wasserstoffatom, einen C₁₋₃-Alkyl- oder C₅₋₁₀-Arylrest bedeutet, die gegebenenfalls mit einem oder mehreren Resten substituiert sind, die unabhängig voneinander ausgewählt sind aus Halogenatomen, Heterocyclen, die 0 bis 10 Kohlenstoffatome und 1 bis 10 Heteroatome, ausgewählt aus Schwefel, Stickstoff und Sauerstoffatomen, enthalten und funktionellen Gruppen und
M⁻ ein Gegenion darstellt.

3. Verfahren nach Anspruch 1 oder 2, umfassend den Schritt:
Herstellung einer Verbindung der Formel III 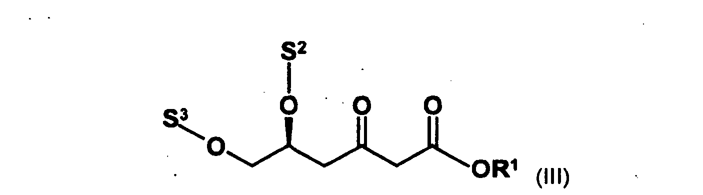 durch Kettenverlängerung einer Verbindung der Formel IV

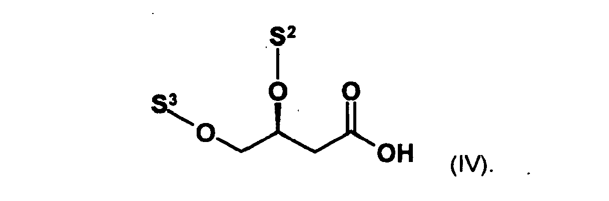

4. Verfahren nach einem der Ansprüche 1 bis 3, wobei die Verbindung der Formel I durch einen der folgenden Verfahrensschritte und anschließend gegebenenfalls durch Öffnung des Lactonringes und gegebenenfalls durch Entfernung von Schutzgruppen in das Statin überführt wird:
a) Umsetzen einer Verbindung der Formel (I) 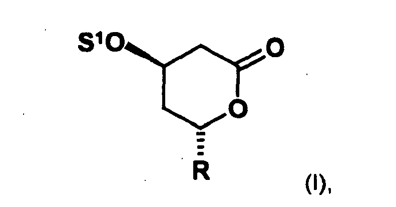 in der der Rest R eine CHO-Gruppe darstellt und der Rest S¹ wie in Anspruch 1 definiert ist, mit einer Verbindung der Formel 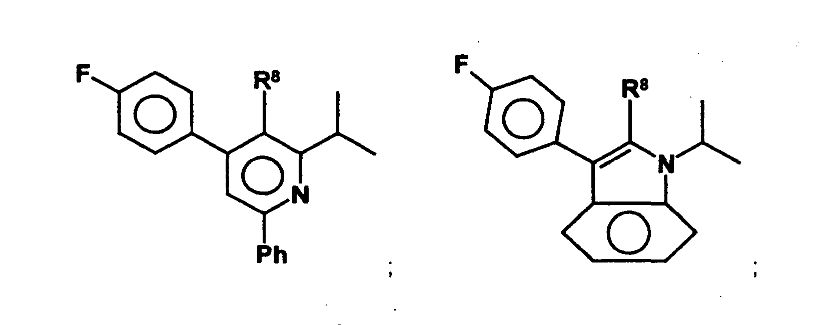 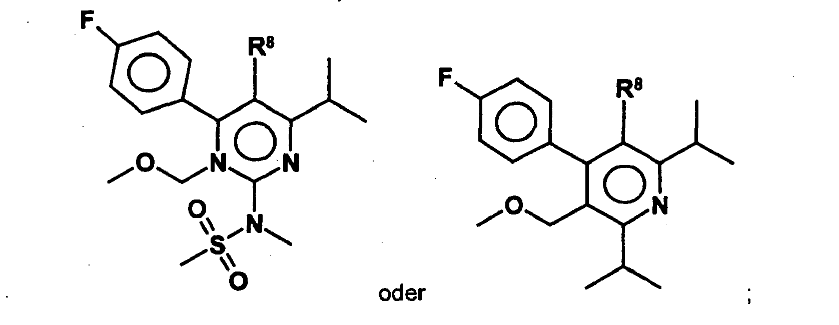 in der
R⁸ -CH=P(R³)₃, -CH₂-P⁺(R³)₃M⁻, 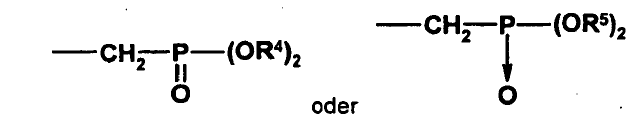 bedeutet, wobei R³, R⁴, R⁵ und M wie in Anspruch 1 definiert sind,
b) Umsetzen einer Verbindung der Formel I 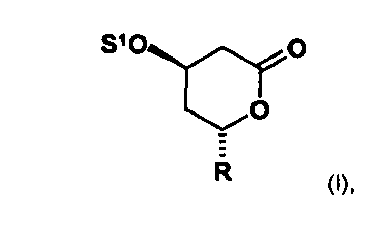 in der
der Rest R -CH=P(R³)₃, -CH₂-P⁺(R³)₃M⁻, 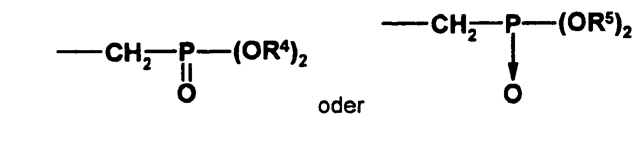 bedeutet,
mit einer Verbindung der Formel 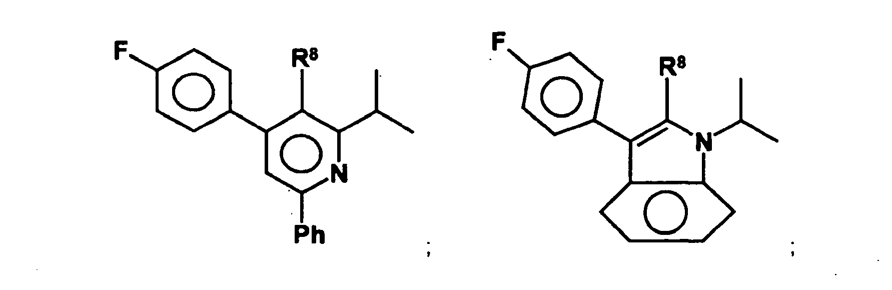 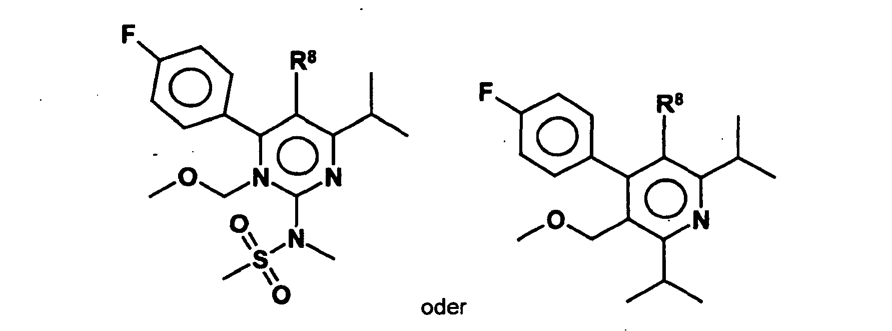 in der
R⁸ -CHO bedeutet,
wobei R³, R⁴, R⁵ und M wie in Anspruch 1 definiert sind,
c) Umsetzen einer Verbindung der Formel I 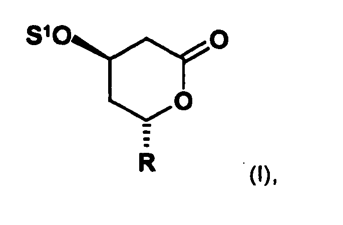 in der
der Rest R eine Gruppe -CH₂-CΞN ist,
Hydrierung der Verbindung der Formel I, in der der Rest R eine Gruppe -CH₂-CΞN ist, zu einer Verbindung der Formel I, in der der Rest R eine Gruppe -CH₂-CH₂NH₂ ist,
und Umsetzung der Verbindung der Formel I, in der der Rest R eine Gruppe -CH₂-CH₂NH₂ ist, mit einer Verbindung der Formel V 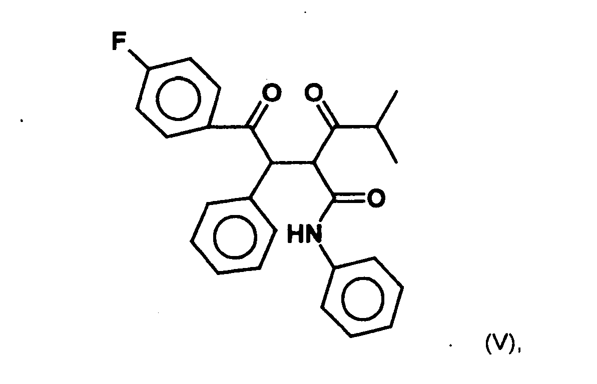
d) Hydrierung einer Verbindung der Formel I 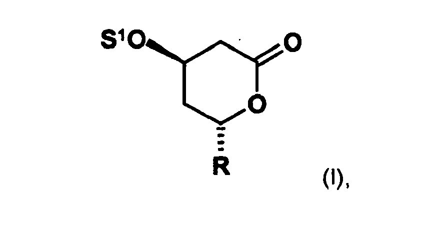 in der
der Rest R eine Gruppe -CH₂-CΞN ist, zu einer Verbindung der Formel I, in der der Rest R eine Gruppe -CH₂-CH₂NH₂ ist,
und Umsetzung der Verbindung der Formel I, in der der Rest R eine Gruppe -CH₂-CH₂NH₂ ist, mit einer Verbindung der Formel V 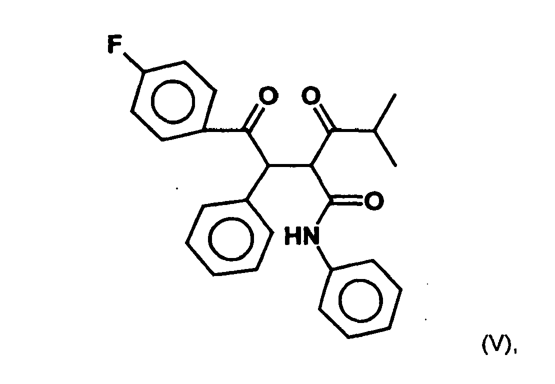
e) Umsetzen einer Verbindung der Formel (I) 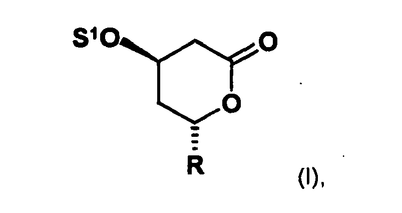 in der
der Rest R eine Gruppe -CH₂-CH₂NH₂ ist, mit einer Verbindung der Formel V

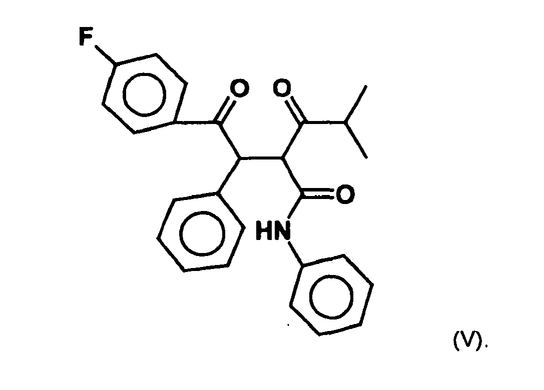

5. Verfahren nach einem der Ansprüche 1 bis 4, **dadurch gekennzeichnet, daß** eine Verbindung der Formel 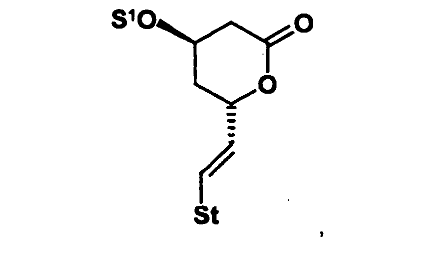 in der S¹ wie in Anspruch 1 definiert ist und St für den Rest des Statins steht, durch katalytische Hydrierung in eine Verbindung der Formel 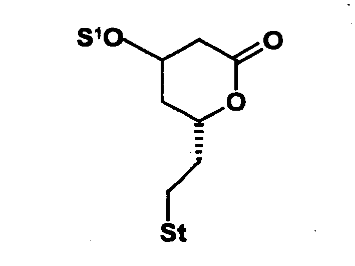 überführt wird und gegebenenfalls die Schutzgruppe S¹ entfernt und gegebenenfalls der Lactonring geöffnet wird.

6. Verfahren nach einem der Ansprüche 1 bis 5, wobei die Hydroxylschutzgruppe S¹ aus einer Trimethylsilyl-, Triisopropylsilyl-, Trimethylsilylethyl-, tert.-Butyldimethylsilyl, tert.-Butylmethylsilyl-, Di-tert-butylmethylsilyl-, tert-Butyldiphenylsilyl-, Triphenylsilyl-, Diphenylmethylsilyl-, Tris(trimethylsilyl)- und para-Tosylschutzgruppe ausgewählt ist.

7. Verfahren nach Anspruch 1, wobei die Schutzgruppen S² und S³ gemeinsam eine Isopropylidenschutzgruppe darstellen.

8. Verfahren nach einem der Ansprüche 2 bis 6, wobei der Rest R ein Rest CH₂R² und R² eine Abgangsgruppe darstellt, wobei die Abgangsgruppe ausgewählt ist aus einem Halogenatom, einem Rest -OSO₂-C₁-C₆-Alkyl oder -OSO₂-C₅-C₁₀-Aryl.

9. Verfahren nach einem der Ansprüche 1 bis 8, wobei der Rest R¹ ein Wasserstoffatom, einen C₁₋₃-Alkyl- oder C₄₋₁₀-Arylrest bedeutet, die gegebenenfalls mit einem oder mehreren Resten substituiert sind, die unabhängig voneinander ausgewählt sind aus Halogenatomen, Heterocyclen, die 0 bis 10 Kohlenstoffatome und 1 bis 10 Heteroatome, ausgewählt aus Schwefel, Stickstoff und Sauerstoffatomen, aufweisen, und funktionellen Gruppen.

10. Verfahren nach einem der Ansprüche 2 bis 9, wobei
R³ einen C₅- bis C₁₀-Arylrest, der gegebenenfalls mit ein oder zwei C₁-C₄-Alkylresten und/oder Halogenatomen substituiert ist, einen C₁-C₄-Alkylrest oder einen C₅-C₁₀-Cycloalkylrest bedeutet,
R⁴ einen C₁-C₄-Alkylrest bedeutet,
R⁵ einen C₁-C₆-Alkyl- oder C₅-C₁₀-Arylrest bedeutet.

11. Verfahren nach einem der Ansprüche 1 bis 10, wobei das Statin Fluvastatin, Rosuvastatin, Cerivastatin, Glenvastatin oder Atorvastatin ist.

## Claims

1. Process for the preparation of a statin, comprising the following steps:
a) Preparation of a compound of the formula II 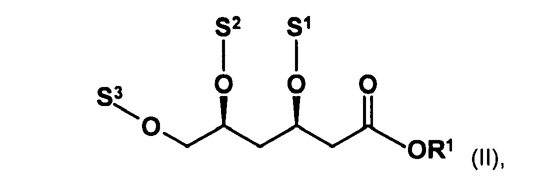 in which
S¹ denotes a hydrogen atom or a hydroxyl protective group,
S² and S³, independently of one another, denote hydroxyl protective groups, wherein the protective groups S² and S³ are bridged, and
R¹ represents a hydrogen atom or a carboxyl protective group,
by stereoselective hydrogenation of a compound of the formula III 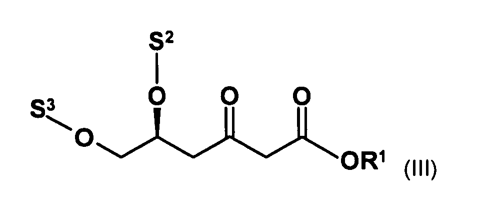 to give a compound of the formula II-a 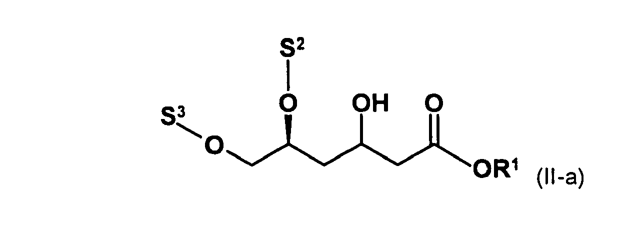 and optionally introduction of a hydroxyl protective group and
b) lactonization of the compound of the formula II to give a compound of the formula I-a

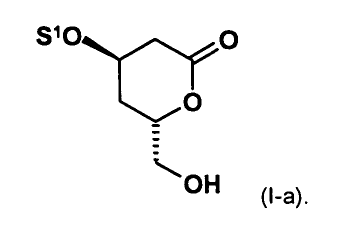

2. Process according to Claim 1, comprising the further step
c) conversion of the compound of the formula I-a 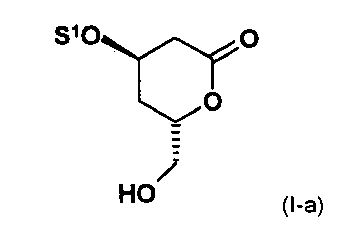
into a compound of the formula I 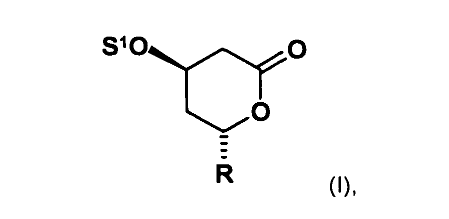 where the radical
S¹ is as defined in Claim 1,
R denotes -CH₂R², -CHO, -CH=P(R³)₃, -CH₂-P⁺(R³)₃M⁻, 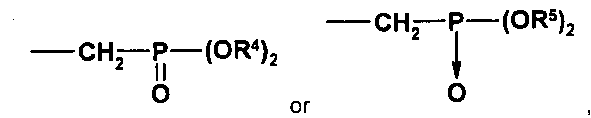
R² denotes a halogen atom, -CΞN, -CH₂NH₂, -SO₂-R⁶ or a leaving group,
R³, R⁴ and R⁵ complete a Wittig radical or a Horner-Wittig radical,
R⁶ denotes a hydrogen atom, a C₁₋₃-alkyl or a C₅₋₁₀-aryl radical, which are optionally substituted by one or more radicals which, independently of one another, are selected from halogen atoms, heterocycles which contain 0 to 10 carbon atoms and 1 to 10 heteroatoms selected from sulphur, nitrogen and oxygen atoms, and functional groups and
M⁻ represents an opposite ion.

3. Process according to Claim 1 or 2, comprising the step:
preparation of a compound of the formula III 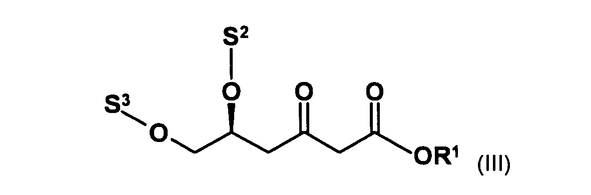 by chain extension of a compound of the formula IV

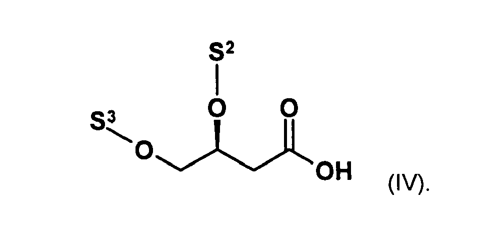

4. Process according to any of Claims 1 to 3, the compound of the formula I being converted into the statin by one of the following process steps and then optionally by opening of the lactone ring and optionally by removal of protective groups:
a) reaction of a compound of the formula (I) 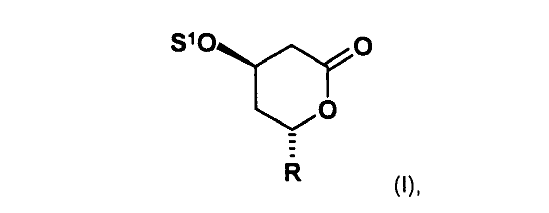 in which the radical R represents a CHO group and the radical S¹ is as defined in Claim 1, with a compound of the formula 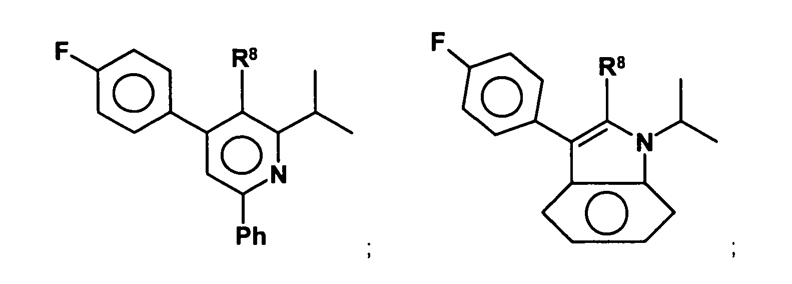 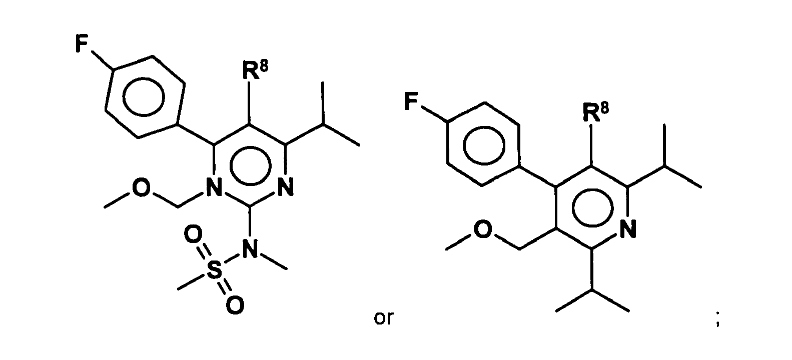 in which
R⁸ denotes -CH=P(R³)₃, -CH₂-P⁺(R³)₃M⁻, 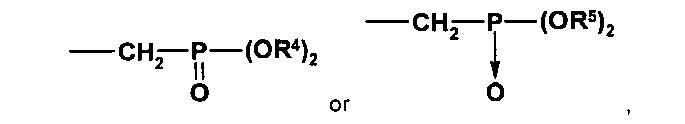
where R³, R⁴, R⁵ and M are as defined in Claim 1,
b) reaction of a compound of the formula I 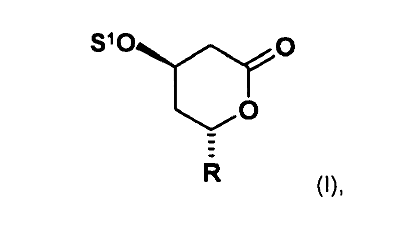 in which
the radical R denotes -CH=P(R³)₃, -CH₂-P⁺(R³)₃M⁻, 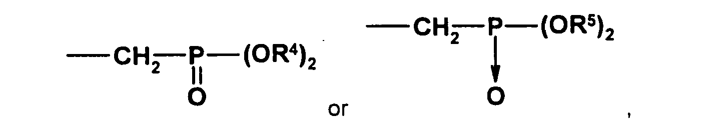 with a compound of the formula 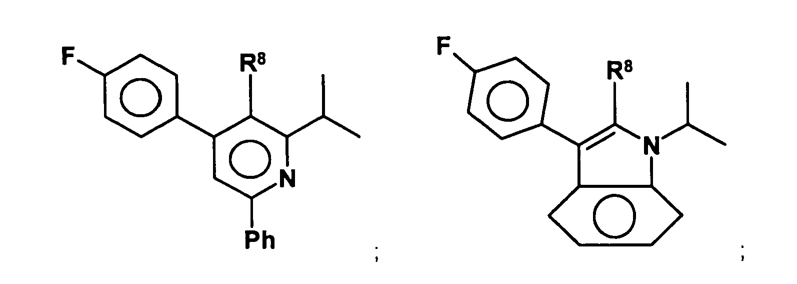 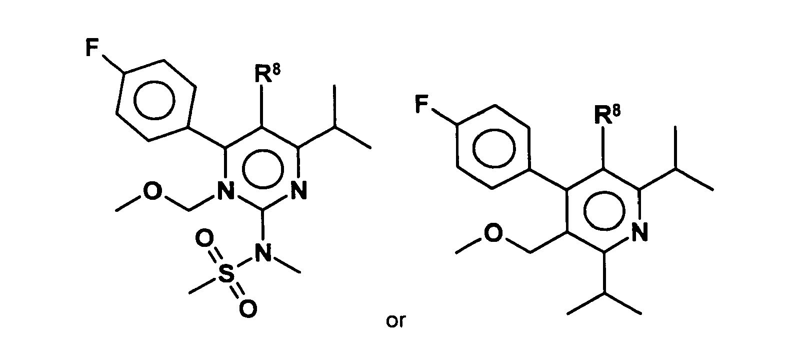 in which
R⁸ denotes -CHO,
where R³, R⁴, R⁵ and M are as defined in Claim 1,
c) reaction of a compound of the formula I 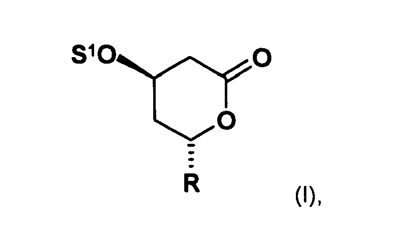 in which
the radical R is a group -CH₂-CΞN,
hydrogenation of the compound of the formula I in which the radical R is a group -CH₂-CΞN, to give a compound of the formula I in which the radical R is a group -CH₂-CH₂NH₂,
and reaction of the compound of the formula I in which the radical R is a group -CH₂-CH₂NH₂ with a compound of the formula V 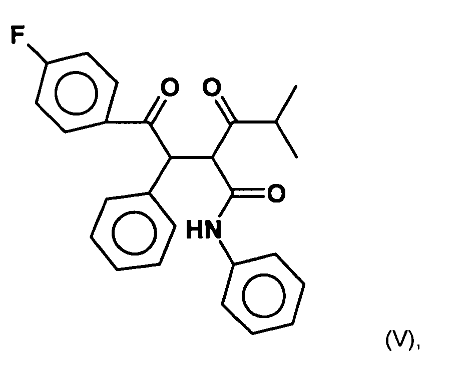
d) hydrogenation of a compound of the formula I 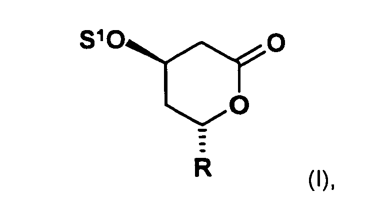 in which
the radical R is a group -CH₂-CΞN, to give a compound of the formula I in which the radical R is a group -CH₂-CH₂NH₂,
and reaction of the compound of the formula I in which the radical R is a group -CH₂-CH₂NH₂ with a compound of the formula V 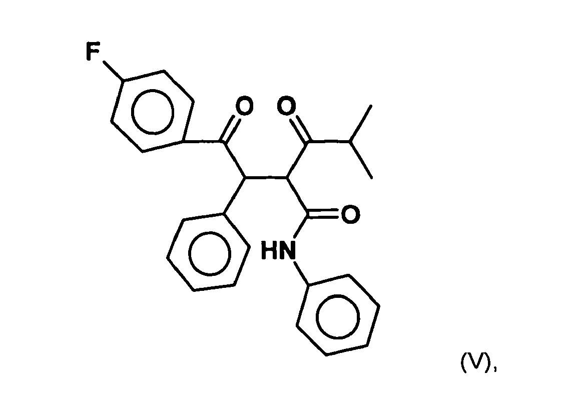
e) reaction of a compound of the formula (1) 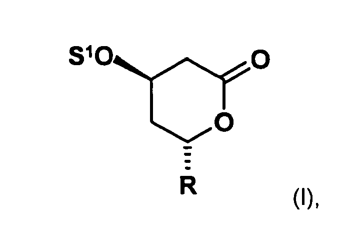 in which
the radical R is a group -CH₂-CH₂NH₂, with a compound of the formula V

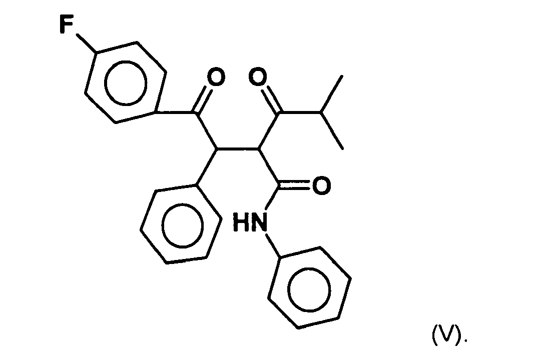

5. Process according any of Claims 1 to 4, **characterized in that** a compound of the formula 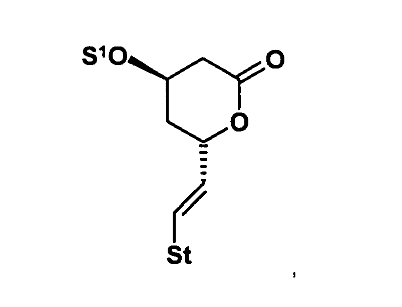 in which S¹ is as defined in Claim 1 and St represents the radical of the statin, is converted into a compound of the formula 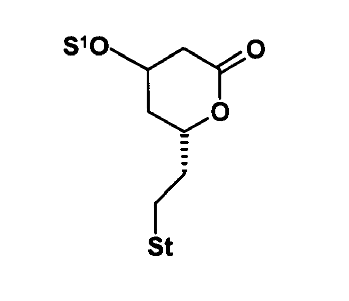 by catalytic hydrogenation, and optionally the protective group S¹ is removed and optionally the lactone ring is opened.

6. Process according to any of Claims 1 to 5, the hydroxyl protective group S¹ being selected from a trimethylsilyl, triisopropylsilyl, trimethylsilylethyl, tert-butyldimethylsilyl, tert-butylmethylsilyl, di-tert-butylmethylsilyl, tert-butyldiphenylsilyl, triphenylsilyl, diphenylmethylsilyl, tris(trimethylsilyl) and para-tosyl protective group.

7. Process according to Claim 6, the protective groups S² and S³ together representing an isopropylidene protective group.

8. Process according to any of Claims 2 to 6, the radical R representing a radical CH₂R² and R² representing a leaving group, the leaving group being selected from a halogen atom and a radical -OSO₂-C₁-C₆-alkyl or -OSO₂-C₅-C₁₀-aryl.

9. Process according to any of Claims 1 to 8, the radical R¹ denoting a hydrogen atom or a C₁₋₃-alkyl or C₄₋₁₀-aryl radical, which are optionally substituted by one or more radicals, which, independently of one another, are selected from halogen atoms, heterocycles which have 0 to 10 carbon atoms and 1 to 10 heteroatoms selected from sulphur, nitrogen and oxygen atoms, and functional groups.

10. Process according to any of Claims 2 to 9,
R³ denoting a C₅- to C₁₀-aryl radical which is optionally substituted by one or two C₁-C₄-alkyl radicals and/or halogen atoms, a C₁-C₄-alkyl radical or a C₅-C₁₀-cycloalkyl radical,
R⁴ denoting a C₁-C₄-alkyl radical,
R⁵ denoting a C₁-C₆-alkyl or C₅-C₁₀-aryl radical.

11. Process according to any of Claims 1 to 10, the statin being fluvastatin, rosuvastatin, cerivastatin, glenvastatin or atorvastatin.

## Revendications

1. Procédé de fabrication d'une statine, comprenant les étapes suivantes :
a) la fabrication d'un composé de formule II suivants : 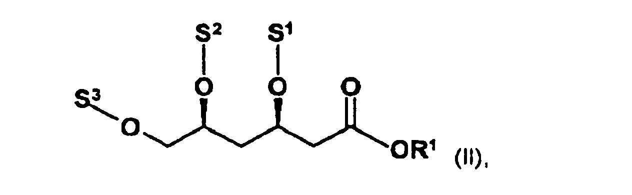 dans laquelle
S¹ S² et S³ représente un atome d'hydrogène ou un groupement protecteur d'hydroxyle, représentent, indépendamment l'un de l'autre, des groupements protecteurs d'hydroxyle, les groupements protecteurs S² et S³ étant pontés, et
R¹ représente un atome d'hydrogène ou un groupement protecteur de carboxyle,
par hydrogénation stéréosélective d'un composé de formule III suivante : 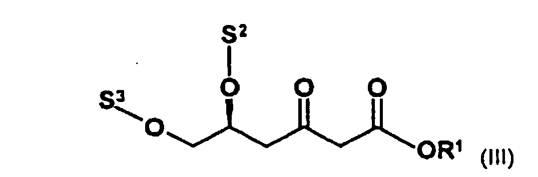 pour donner un composé de formule II-a suivante : 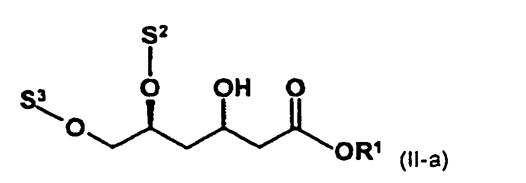 et éventuellement l'introduction d'un groupement protecteur d'hydroxyle et
b) la lactonisation du composé de formule II pour donner un composé de formule I-a suivante :

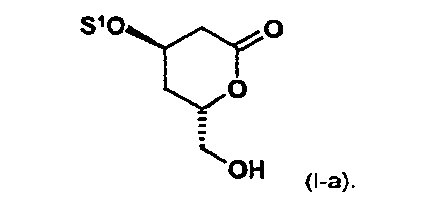

2. Procédé selon la revendication 1, comprenant l'étape supplémentaire consistant à :
c) transformer le composé de formule I-a suivante : 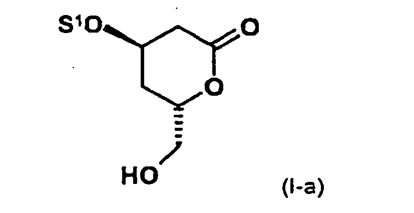 en un composé de formule I suivante : 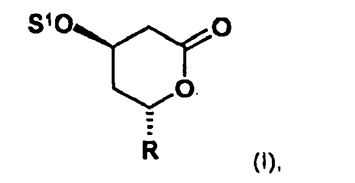 dans laquelle le radical
S¹ est tel que défini dans la revendication 1,
R représente -CH₂R², -CHO, -CH=P(R³)₃, -CH₂-P⁺(R³)₃M⁻, 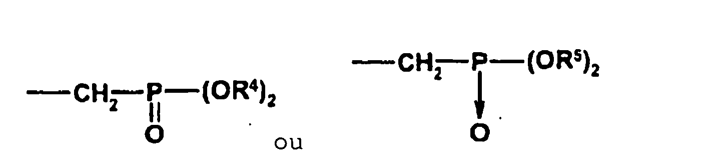
R² représente un atome d'halogène, -C=N, -CH₂NH₂, -SO₂-R⁶ ou un groupement partant,
R³, R⁴ et R⁵ complètent un radical de Wittig ou un radical de Horner-Wittig,
R⁶ représente un atome d'hydrogène, un radical alkyle en C₁₋₃ ou un radical aryle en C₅₋₁₀, qui sont substitués éventuellement par un ou plusieurs radicaux, qui sont choisis indépendamment l'un de l'autre parmi des atomes d'halogène, des hétérocycles, qui contiennent 0 à 10 atomes de carbone et 1 à 10 hétéroatomes, choisis parmi le soufre, l'azote et des atomes d'oxygène, et des groupements fonctionnels et
M⁻ représente un contre-ion.

3. Procédé selon la revendication 1 ou 2, comprenant l'étape consistant à :
fabriquer un composé de formule III suivante : 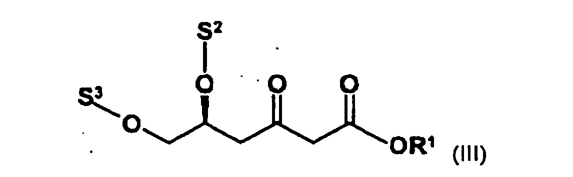
par allongement de chaîne d'un composé de formule IV suivante :

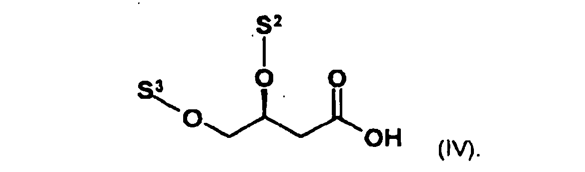

4. Procédé selon l'une quelconque des revendications 1 à 3, dans lequel le composé de formule I est transformé en statine par l'une des étapes de procédé suivantes, puis éventuellement par l'ouverture du cycle de lactone et, éventuellement, par l'élimination de groupements protecteurs :
a) réaction d'un composé de formule (I) suivante : 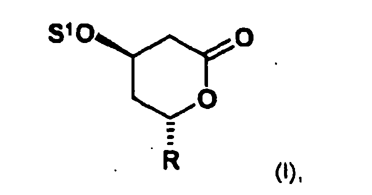 dans laquelle le radical R est un groupement CHO et le radical S¹ est tel que défini dans la revendication 1, avec un composé de formule suivante : 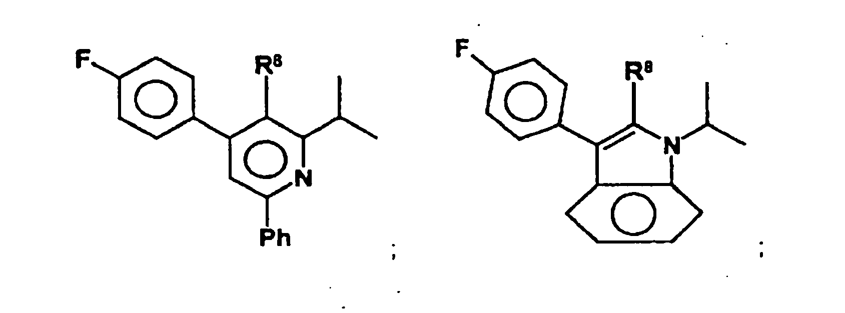 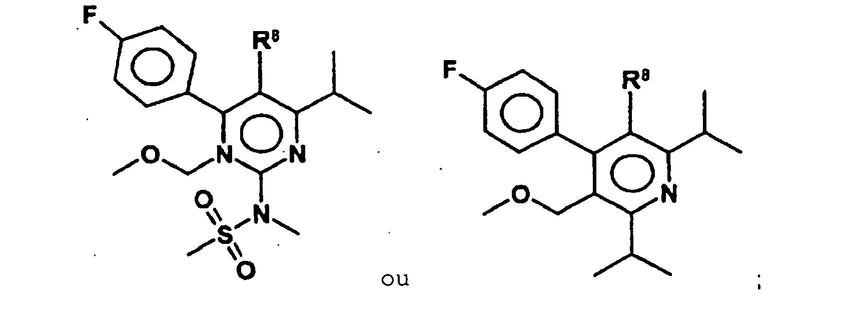 dans laquelle
R⁸ représente -CH=P(R³)₃, -CH₂-P⁺(R³)₃M⁻, 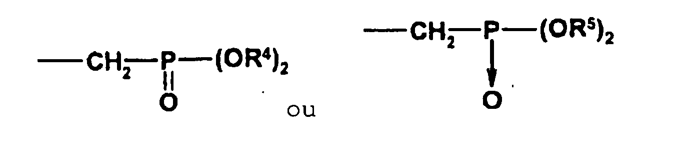 où R³, R⁴, R⁵ et M sont tels que définis dans la revendication 1,
b) la réaction d'un composé de formule I suivante : 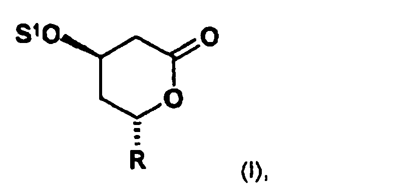 dans laquelle
le radical R représente -CH=P(R³)₃, -CH₂-P⁺(R³)₃M⁻, 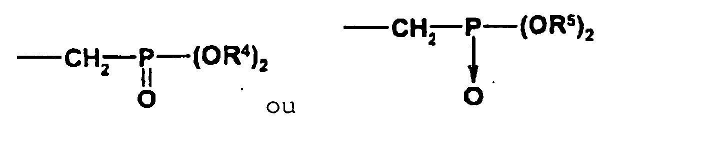 avec un composé de formule suivante : 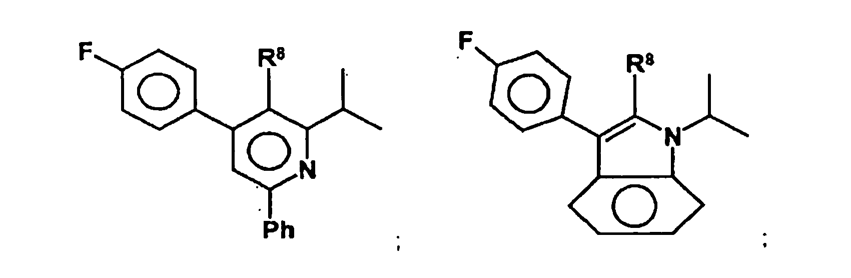 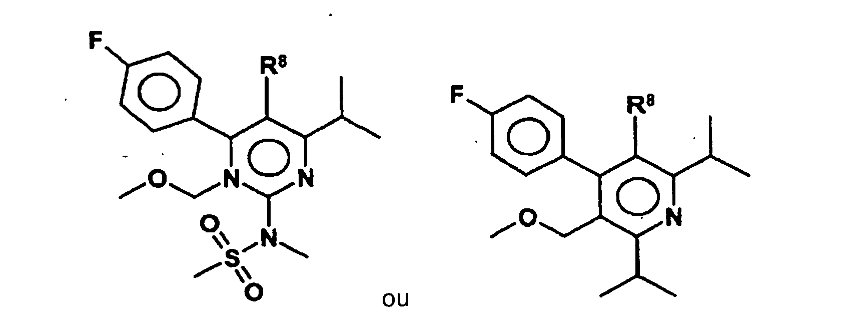 dans laquelle
R⁸ représente -CHO,
où R³, R⁴, R⁵ et M sont tels que définis dans la revendication 1,
c) la réaction d'un composé de formule I suivants : 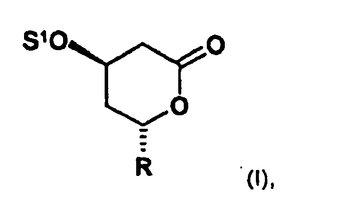 dans laquelle
le radical R est un groupement -CH₂-C≡N, l'hydrogénation du composé de formule I, dans laquelle le radical R est un groupement -CH₂-C≡N, pour donner un composé de formule I, dans laquelle le radical R est un groupement -CH₂-CH₂NH₂,
et la réaction du composé de formule I, dans laquelle le radical R est un groupement -CH₂-CH₂NH₂, avec un composé de formule V suivants : 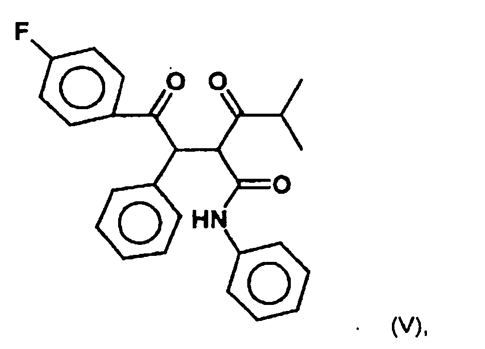
d) l'hydrogénation d'un composé de formule I suivants : 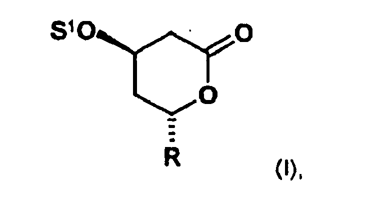 dans laquelle le radical R est un groupement -CH₂-C≡N, pour donner un composé de formule I, dans laquelle le radical R est un groupement -CH₂-CH₂-NH₂,
et la réaction du composé de formule I, dans laquelle le radical R est un groupement -CH₂-CH₂NH₂, avec un composé de formule V suivante : 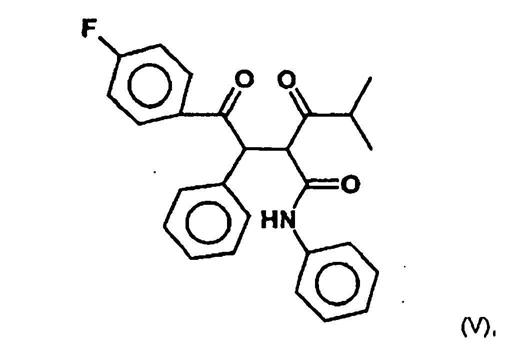
e) la réaction d'un composé de formule (I) suivante : 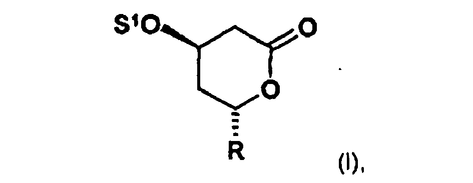 dans laquelle
le radical R est un groupement -CH₂-CH₂NH₂, avec un composé de formule V suivante :

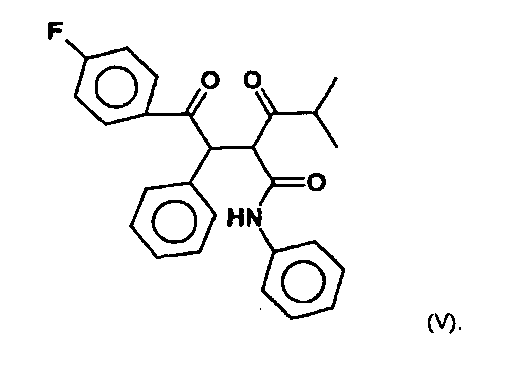

5. Procédé selon l'une quelconque des revendications 1 à 4, **caractérisé en ce que** l'on transforme un composé de formule suivante : 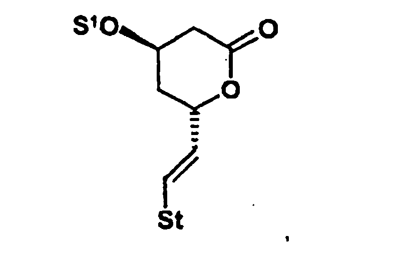 dans laquelle S¹ est tel que défini dans la revendication 1 et St représente le radical de la statine, par hydrogénation catalytique en un composé de formule suivante : 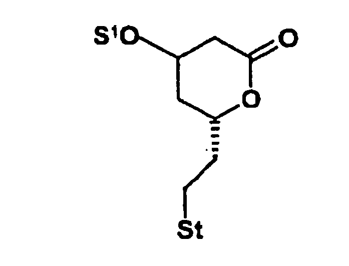 et **en ce que** l'on élimine éventuellement le groupement protecteur S¹ et que l'on ouvre éventuellement le cycle de lactone.

6. Procédé selon l'une quelconque des revendications 1 à 5, dans lequel le groupement protecteur d'hydroxyle S¹ est choisi parmi un groupement protecteur de type triméthylsilyle, triisopropylsilyle, triméthylsilyléthyle, tert-butyldiméthylsilyle, tert-butylméthylsilyle, di-tert-butylméthylsilyle, tert-butyldiphénylsilyle, triphénylsilyle, diphénylméthylsilyle, tris(triméthylsilyle) et para-tosyle.

7. Procédé selon la revendication 1, dans lequel les groupements protecteurs S² et S³ représentent ensemble un groupement protecteur de type isopropylidène.

8. Procédé selon l'une quelconque des revendications 2 à 6, dans lequel le radical R représente un radical CH₂R² et R² représente un groupement partant, dans lequel le groupement partant est choisi parmi un atome d'halogène, un radical -OSO₂-alkyle en C₁-C₆ ou -OSO₂-aryle en C₅-C₁₀.

9. Procédé selon l'une quelconque des revendications 1 à 8, dans lequel le radical R¹ représente un atome d'hydrogène, un radical alkyle en C₁₋₃ ou un radical aryle en C₄₋₁₀, qui sont substitués éventuellement par un ou plusieurs radicaux, qui sont choisis indépendamment l'un de l'autre parmi des atomes d'halogène, des hétérocycles, qui comportent 0 à 10 atomes de carbone et 1 à 10 hétéroatomes choisis parmi le soufre, l'azote et des atomes d'oxygène, et des groupements fonctionnels.

10. Procédé selon l'une quelconque des revendications 2
à 9, dans lequel
R³ représente un radical aryle en C₅-C₁₀, qui est substitué éventuellement par un ou deux radicaux alkyle en C₁-C₄ et/ou par des atomes d'halogène, un radical alkyle en C₁-C₄ ou un radical cycloalkyle en C₅-C₁₀,
R⁴ représente un radical alkyle en C₁-C₄,
R⁵ représente un radical alkyle en C₁-C₆ ou un radical aryle en C₅-C₁₀.

11. Procédé selon l'une quelconque des revendications 1 à 10, dans lequel la statine est la fluvastatine, la rosuvastatine, la cérivastatine, la glenvastatine ou l'atorvastatine.
